# EUROPEAN PATENT APPLICATION

(11) **EP 4 703 726 A1**
(43) Date of publication of application: **04.03.2026**
(21) Application number: 24797124.5
(22) Date of filing: 25.04.2024
(51) Int. Cl.: G01N 33/53, C12M 1/34, C12Q 1/06, G01N 33/48, G01N 33/50

(54) **METHOD FOR MEASURING RELATIVE AMOUNT OF SPECIFIC CELL SUBPOPULATION IN CD4+ T-CELL POPULATION**

(30) Priority: 26.04.2023 JP 2023072026
(71) Applicant: Immunit Research Inc., Tokyo 160-0022 (JP)
(72) Inventor: KENMOCHI, Megumi, Tokyo 160-0022 (JP); YAMASHIRO, Tomoko, Tokyo 160-0022 (JP); AMETANI, Akio, Tokyo 160-0022 (JP)
(74) Representative: Murgitroyd & Company
(86) International application number: PCT/JP2024/016252
(87) International publication number: WO 2024/225379

(57) **Abstract**

[Problem]

To provide: a method for more efficiently measuring the relative amount of a specific cell subpopulation in a CD4⁺ T cell population; and a kit for the method. [Solution] The present invention provides a method for determining the relative amount of a CCR4⁻CCR6⁺ cell subpopulation in a CD4⁺ T-cell population in a sample derived from a subject, the method comprising: a step for selecting a CXCR3⁻ cell subpopulation in the CD4⁺ T-cell population; a step for setting a boundary between a CCR4⁻ cell subpopulation and a CCR4⁺ cell subpopulation in the CXCR3⁻ cell subpopulation; and a step for measuring the relative amount of the CCR4⁻ CCR6⁺ cell subpopulation in the CD4⁺ T-cell population by using the boundary.

## Description

### [Technical Field]

The present invention relates to measurement of the relative amount of a medically important cell subpopulation in a CD4⁺ T cell population.

### [Background Art]

It is known that there are several medically significant cell populations in a CD4⁺ T cell population. For example, the relative amount of a CD4⁺CD62L^{low} cell subpopulation or a CCR4⁻CCR6⁺ cell subpopulation is particularly useful for the response to cancer therapy and is medically significant (Patent Literature 1 and Patent Literature 2).

These cell subpopulations are typically separated by collecting 8 ml or more of peripheral blood, separating and freezing PBMCs (peripheral blood mononuclear cells) using a centrifuge, etc. by a laboratory technician at the venue of blood collection, transporting the frozen cells to a measurement laboratory, storing the frozen cells after transportation as needed, then culturing and staining the cells, and subjecting the cells to measurement by FACS.

### [Citation List]

### [Patent Literature]

[PTL 1] International Publication No. WO 2018/147291
[PTL 2] International Publication No. WO 2022/054796

### [Summary of Invention]

### [Technical Problem]

The problem to be solved by the present invention is to provide methods for more efficiently measuring the relative amount of a specific cell subpopulation in a CD4⁺ T cell population and kits therefor.

### [Solution to Problem]

The inventors have found that it is advantageous to initially separate CXCR3⁺/⁻ cell groups in order to measure the relative amount of a CCR4⁻CCR6⁺ cell subpopulation in a CD4⁺ T cell population.

The inventors also found preferred sample storage conditions for successfully measuring the relative amount of a specific cell subpopulation in a CD4⁺ T cell population with a whole blood sample.

The present invention provides, for example, the following items.

### (Item 1)

A method of determining a relative amount of a CCR4⁻CCR6⁺ cell subpopulation in a CD4⁺ T cell population in a sample derived from a subject, comprising:
selecting a CXCR3⁻ cell subpopulation in the CD4⁺ T cell population;
setting a boundary between a CCR4⁻ cell subpopulation and a CCR4⁺ cell subpopulation in the CXCR3⁻ cell subpopulation; and
measuring a relative amount of a CCR4⁻CCR6⁺ cell subpopulation in a CD4⁺ T cell population by using the boundary.

### (Item 2)

The method of item 1, wherein the sample is a whole blood sample or a peripheral blood mononuclear cell (PBMC) sample of peripheral blood of the subject.

### (Item 3)

The method of item 2, wherein the sample is a whole blood sample.

### (Item 4)

The method of item 3, wherein the sample has been stored for one day or longer after collection from the subject.

### (Item 5)

The method of item 4, wherein the sample has been stored for one to six days after collection from the subject.

### (Item 6)

The method of item 4, wherein the sample has been stored for one to three days after collection from the subject.

### (Item 7)

The method of item 4, wherein the sample has been stored at about 10°C to about 40°C.

### (Item 8)

The method of item 4, wherein the sample has been stored at about 12°C to about 25°C.

### (Item 9)

The method of item 3, wherein the sample is about 10 µl to about 500 µl.

### (Item 10)

A method of determining a relative amount of a CCR4⁻CCR6⁺ cell subpopulation in a CD4⁺ T cell population in a sample derived from a subject, comprising:
preparing a whole blood sample of the subject; and
measuring a relative amount of a CCR4⁻CCR6⁺ cell subpopulation in a CD4⁺ T cell population in the whole blood sample.

### (Item 11)

The method of item 10, wherein the sample has been stored for one day or longer after collection from the subject.

### (Item 12)

The method of item 11, wherein the sample has been stored for one to six days after collection from the subject.

### (Item 13)

The method of item 11, wherein the sample has been stored for one to three days after collection from the subject.

### (Item 14)

The method of item 11, wherein the sample has been stored at about 10°C to about 40°C.

### (Item 15)

The method of item 11, wherein the sample has been stored at about 12°C to about 25°C.

### (Item 16)

The method of item 10, wherein the sample is about 10 µl to about 500 µl.

### (Item 17)

A kit for use in the method of any one of items 1-16, comprising a detecting agent for CD4, a detecting agent for CCR4, and a detecting agent for CCR6.

### (Item 18)

The kit of item 17, further comprising a detecting agent for CXCR3.

### (Item 19)

The kit of item 16, wherein the detecting agent is an antibody.

### [Advantageous Effects of Invention]

The present invention enables a more accurate measurement of a medically significant cell subpopulation (e.g., a CCR4⁻CCR6⁺ cell subpopulation or a CD62L^{low} cell subpopulation) in a CD4⁺ T cell population.

The problem of a blood collection venue being limited (a centrifuge, etc. is available and a technician is present) and the problem of a significant difference between personnel/institutions who are and are not good at separating PBMCs can be solved, thus allowing a sample to be obtained with low variability between facilities without effort at a blood collection venue.

### [Brief Description of Drawings]

[Figure 1] Figure **1** shows histograms of CXCR3 and scatter plots of CCR4 vs CCR6 for whole blood and PBMCs.
[Figure 2] Figure **2** shows histograms of CXCR3 and scatter plots of CCR4 vs CCR6 from changing the storage period of whole blood.
[Figure 3] Figure **3** shows scatter plots of CD4 vs CD8 when stored by changing the temperature and number of days.
[Figure 4] Figure **4** shows histograms of CD62L for CD4⁺ cells when stored by changing the temperature and number of days.
[Figure 5] Figure **5** shows histograms of CD62L for CD8⁺ cells when stored by changing the temperature and number of days.
[Figure 6] Figure **6** shows Th7R when stored by changing the temperature and number of days.
[Figure 7] Figure **7** shows scatter plots of CCR7 and CD45RA for CD4⁺ cells when stored by changing the temperature and number of days.
[Figure 8] Figure **8** shows scatter plots of CCR7 and CD45RA for CD8⁺ cells when stored by changing the temperature and number of days.
[Figure 9] Figure **9** shows a comparison of measurements of a sample of separating and cryopreserving PBMCs from stored whole blood and of whole blood.
[Figure 10] Figure **10** shows diagrams comparing expression of CCR4, CCR6, and CXCR3 of CD4⁺ cells for each storage method.
[Figure 11A] Figure **11A** shows CCR6 vs CCR4 scatter plots for CD4⁺ cells, without showing a gate.
[Figure 11B] Figure **11B** shows CCR6 vs CCR4 scatter plots for CD4⁺CXCR3⁻ cells, without showing a gate.
[Figure 11C] Figure **11C** shows CCR6 vs CCR4 scatter plots for CD4⁺ cells, with the gate shown.
[Figure 11D] Figure **11D** shows CCR6 vs CCR4 scatter plots for CD4⁺CXCR3⁻ cells, with the gate shown.
[Figure 12] Figure **12** shows results of WB panel 3 staining of self-collected minute blood samples.
[Figure 13] Figure **13** shows a schematic diagram representing Th differentiation of human CD4 T cells.
[Figure 14] Figure **14** shows peripheral blood CD4⁺ T cell clusters and a schematic diagram showing chemokine receptors expressed in each cluster.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", etc. in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions of the terms and/or the detailed basic technology that are particularly used herein are described hereinafter as appropriate.

As used herein, "about" refers to a range of ± 10% from the numerical value that is described subsequent to "about".

As used herein, "biomarker" refers to a characteristic that can be objectively measured and evaluated as an indicator of a normal biological process, pathological process, or a pharmacological response to therapeutic intervention.

As used herein, "cancer" refers to malignant tumor, which is highly atypic, expands faster than normal cells, and can destructively infiltrate or metastasize to a surrounding tissue, or the presence thereof. In the present invention, cancer includes solid cancer and hematopoietic tumor. Examples thereof include, but are not limited to, non-small cell lung cancer, small cell lung cancer, renal cancer, Hodgkin's disease, head and neck cancer, breast cancer, gastric cancer, malignant melanoma, colon cancer, acute myeloid leukemia, chronic myeloid leukemia, acute lymphatic leukemia, gastrointestinal stromal tumor, pancreatic neuroendocrine tumor, skin cancer, etc.

As used herein, "cancer immunotherapy" refers to a method of treating cancer using a biological defense mechanism such as the immune mechanism of an organism.

As used herein, "antitumor immune response" refers to any immune response against tumor in an organism.

As used herein, "correlation" refers to two events having a statistically significant correlation. For example, "relative amount of B correlated with A" refers to the relative amount of B being statistically significantly affected (e.g., increase or decrease) when A occurs.

As used herein, "cell subpopulation" refers to any population of cells with some type of common feature in a cell population including cells with diverse characteristics. For cell subpopulations with a specific name that is known in the art, a specific cell subpopulation can be mentioned by using such a term or by describing any property (e.g., expression of a cell surface marker).

As used herein, the term "relative amount" with regard to cells can be interchangeably used with "ratio". Typically, the terms "relative amount" and "ratio" refer to the number of cells constituting a given cell subpopulation (e.g., CCR4⁻ CCR6⁺ cell subpopulation) with respect to the number of cells constituting a specific cell population (e.g., CD4⁺ T cell population).

As used herein, "relative value" refers to a value obtained by calculating a certain value while using another value as a baseline of comparison.

As used herein, the term "detecting agent" broadly refers to any agent that is capable of detecting a substance of interest (e.g., cell surface marker, etc.).

As used herein, the "amount" of a certain cell subpopulation encompasses the absolute number of certain cells and relative amount of a ratio in a cell population.

As used herein, "flow cytometry" refers to a technology of measuring the number of cells, individuals, and other biological particles suspended in a liquid and individual physical/chemical/biological attributes. Results of flow cytometry can be typically expressed as a dot plot, with FSC on the X axis and SSC on the Y axis. Each cell is indicated by a dot (point) in a diagram. The position thereof is determined by the relative value of FSC and SSC. Lymphocytes which have a relatively small size and simple internal structure are displayed on the bottom left section, granulocytes which have a large size and granules inside are displayed on the top right section, and monocytes which have a large size but a simple internal structure are displayed between lymphocytes and granulocytes, with each forming a population separated from one another. The results of flow cytometry can be represented by a histogram, dot plot, etc.

As used herein, "subject" can be any animal, typically a mammal. Examples of mammals include, but are not limited to, livestock animals (e.g., cows, sheep, cats, dogs, and horses), primates (e.g., humans, and non-human primates such as monkeys), rabbits, and rodents (e.g., mice and rats). In a specific embodiment, the subject is a human.

As used herein, "cancer therapy" refers to any therapy administered to a cancer patient for removing or reducing tumor, including, but not limited to, cancer immunotherapy, radiation therapy, molecularly targeted drug therapy, surgical operation, cell infusion, any combination thereof, etc.

### (Th7R)

As used herein, "effector cells" refer to naive cells, which have not been subjected to antigen stimulation, differentiating into immune cells that have actually acquired a function associated with immunity. In particular, three types thereof, i.e., Th1, Th2, and Th17, are mainly known for human helper T cells. Each can release a characteristic cytokine such as interferon γ (IFNγ) or interleukin 17 (IL-17). While each Th subset has a characteristic transcription factor, such as Th1 expressing T-bet, Th2 expressing GATA-3, etc., there are cells that express both T-bet and GATA-3. Such cells are expressed as "Th1/Th2" or "Th1/2". As used herein, "Th1/Th17" or "Th1/17" expresses both T-bet which is characteristic of Th1 and RORyt which is characteristic of Th17.

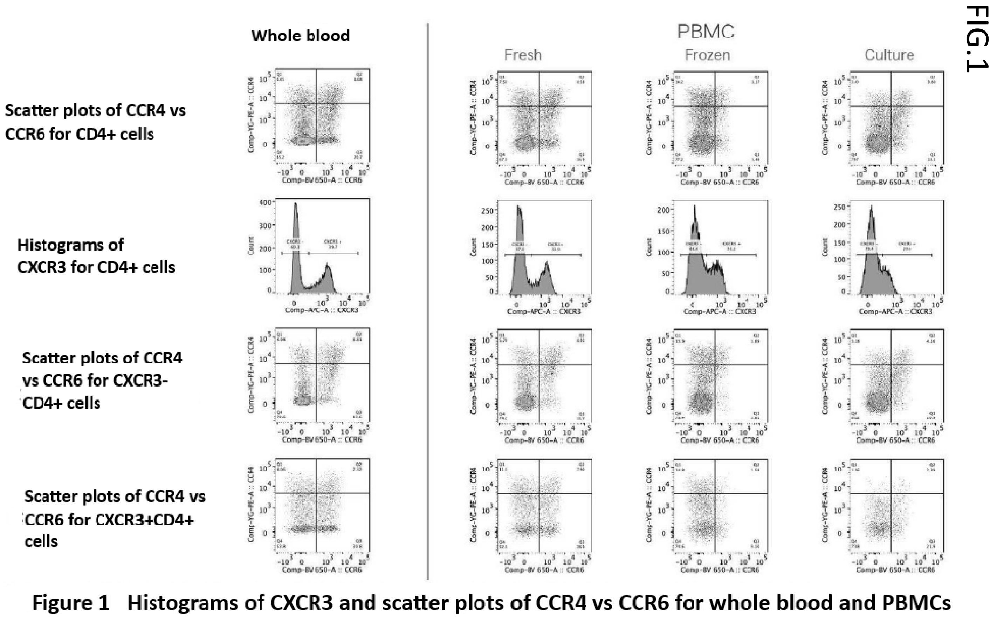

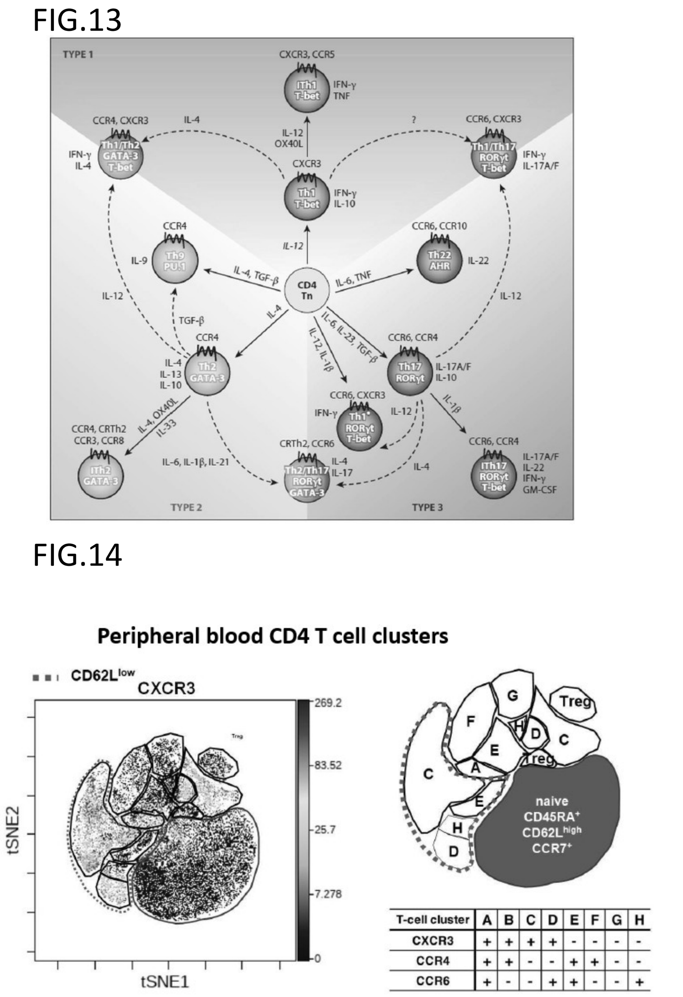

Effector cells such as Th1, Th2, and Th17 can also be classified by the type of chemokine receptor (CCR) that is expressed. For example, Th1 expresses CXCR3, Th2 expresses CCR4, and Th17 expresses CCR4 and CCR6. Classification of Th subsets can be expressed as shown in, for example, Figure **13** (Annual Review of Immunology, Vol. 34:317-334 Heterogeneity of Human CD4+ T Cells Against Microbes, Fig. 1). As used herein, "Th1/Th17" or "Th1/17" expresses CXCR3 which is characteristic of Th1, and CCR6 between the two CCRs characteristic of Th17. As used herein, CCR6 SP (single positive) is a type that was not classified in the past in Th type classification by CCR, and is a Th subset expressing only CCR6 among CCR4, CCR6, and CXCR3. As used herein, TP (triple positive) is a type that was not classified in the past in Th type classification by CCR, and is a Th subset expressing all three of CCR4, CCR6, and CXCR3.

It was found in International Publication No. WO 2022/054796 that Th1/17 and CCR6 SP CD4⁺ T cell clusters among Th clusters exhibit high correlation with progression free survival. Figure **14** summarizes chemokine receptors expressed in each fraction in a diagram (corresponding to Figure 17 in International Publication No. WO 2022/054796). As is apparent from this diagram, the portion surrounded by a dotted line is a CD62L^{low} cell population. It can be understood that there are four clusters (C, D, E, and H) with a different expression of a chemokine receptor therein. Fraction D therein is Th1/17 expressed as CXCR3⁺CCR4⁻CCR6⁺, and fraction H is CCR6 SP expressed as CXCR3⁻CCR4⁻CCR6⁺.

As used herein, "Th7R" is a cell subpopulation combining a Th1/17 cell subpopulation (CD4⁺CXCR3⁺CCR4⁻CCR6⁺) and a CCR6 SP cell subpopulation (CD4⁺CXCR3⁻CCR4⁻CCR6⁺). It is understood that a Th7R cell subpopulation in blood is responsible for the systemic immunity and is a medically significant cell subpopulation, which can be used in, for example, predicting an effect of cancer immunotherapy, especially predicting an effect of an immune checkpoint inhibitor (anti-PD-1 antibody, anti-PD-L1 antibody, anti-CTLA-4 antibody, etc.), predicting a response to cancer therapy such as chemotherapy or therapy using a molecularly targeted drug (e.g., epidermal growth factor receptor (EGFR) tyrosine kinase inhibitor), and is observed in hepatitis B patients, mouse chlamydia lung infection models, opportunistic infection patients, dengue fever patients, acetylcholine antibody-positive myasthenia gravis patients, etc.

In this manner, Th7R is a concept encompassing both a CXCR3⁺ cell subpopulation and a CXCR3⁻ cell subpopulation. Thus, it is not essential in principle to measure whether CXCR3 is expressed when measuring the ratio of Th7R cells in a CD4⁺ cell subpopulation. However, as disclosed herein, gating of CCR4 with respect to a CXCR3⁻ cell subpopulation allowed a gate to be more clearly determined than an embodiment of gating CCR4 with respect to the entire CD4⁺ cells without confirming the expression of CXCR3 (see Example 5). Thus, the present invention elucidated that the following series of steps is preferred when measuring a Th7R cell subpopulation:
1. selecting a CXCR3⁻ cell subpopulation in a CD4⁺ T cell population;
2. setting a boundary (gating) between a CCR4⁻ cell subpopulation and a CCR4⁺ cell subpopulation in a CXCR3⁻ cell subpopulation; and
3. measuring a relative amount of a CCR4⁻CCR6⁺ cell subpopulation in the CD4⁺ T cell population by using the set boundary (gate).

### (Cancer)

Examples of target cancer in the invention include, but are not limited to, melanoma (malignant melanoma), non-small cell lung cancer, renal cell cancer, malignant lymphoma (Hodgkin's or non-Hodgkin's lymphoma), head and neck cancer, urological cancer (bladder cancer, urothelial cancer, and prostate cancer), small cell lung cancer, thymic carcinoma, gastric cancer, esophageal cancer, esophagogastric junction cancer, liver cancer (hepatocellular carcinoma and intrahepatic cholangiocarcinoma), primary brain tumor (glioblastoma and primary central nervous system lymphoma), malignant pleural mesothelioma, gynecologic cancer (ovarian cancer, cervical cancer, and uterine cancer), soft tissue sarcoma, cholangiocarcinoma, multiple myeloma, breast cancer, colon cancer, etc.

### (Whole blood sample)

A sample for fractionation/separation of T cells can be suitably collected from a subject using a conventional method. For example, such a sample can be collected from peripheral blood, bone marrow, tumor tissue, hematopoietic tissue, spleen, normal tissue, lymph, etc. of a subject. Sample collection from peripheral blood can be advantageous for being simple and non-invasive.

In the present invention, a whole blood sample derived from peripheral blood or a PBMC sample prepared therefrom can be used, and a whole blood sample can be preferably used. A whole blood sample can be collected in an anticoagulant-containing blood collection tube. While the present invention can use any anticoagulant known in the art, the anticoagulant can be preferably heparin (heparin sodium or heparin lithium), EDTA, sodium citrate, ACD-A, etc. and more preferably heparin. As shown in the Examples described below, the ratio varied as the number of days of storage in a blood collection tube increased in many cells, when EDTA was used, but Th7R (CD4⁺CCR6⁺CCR4⁻) is characterized in that the ratio of variation is relatively low even when EDTA is used.

As is apparent from the Examples herein, the preferred storage conditions for a whole blood sample can vary depending on the cell marker to be measured. For Th7R, variation in measurement results was low in a broad temperature range, even after long-term storage. Thus, a whole blood sample can be stored at about 10°C to about 40°C, more preferably at about 12°C to about 25°C, when measuring Th7R in CD4⁺ cells. For measurement of Th7R, a whole blood sample can be sufficiently measured any time within one week from blood collection (preferably within 6 days, more preferably 3 days from blood collection) when stored at about 12°C to about 25°C, which is unexpected in comparison to other markers described below. Since whether measurements can be taken immediately after blood collection is dependent on the blood collection venue or blood collection facility, it can be advantageous that measurements can be taken stably even after a day or more has passed, instead of immediately after blood collection. Thus, the storage period of a whole blood sample for measuring Th7R can be, for example, 1 day to 1 week, 1 day to 6 days, 1 day to 3 days, etc. in the present invention.

When measuring CD62L^{low} in CD4⁺ cells, it was found that a higher temperature range than that used for Th7R is preferable. Specifically, a whole blood sample can be stored at about 15°C to about 40°C, more preferably at about 18°C to about 37°C, when measuring CD62L^{low}. For measurement of CD62L^{low}, a whole blood sample can be sufficiently measured any time within one week from blood collection (preferably within 3 days from blood collection) when stored at about 18°C to about 37°C. Since whether measurements can be taken immediately after blood collection is dependent on the blood collection venue or blood collection facility, it can be advantageous that measurements can be taken stably even after a day or more has passed, instead of immediately after blood collection. Thus, the storage period of a whole blood sample for measuring CD62L^{low} in CD4⁺ cells can be, for example, 1 day to 1 week, 1 day to 6 days, 1 day to 3 days, etc.

In a specific embodiment, it can be advantageous to measure the ratio of a Th7R·CD62L^{low} cell subpopulation. In such a case, measurements can be sufficiently taken any time within one week from blood collection (preferably within 3 days from blood collection) by storing at about 18°C to about 25°C. Thus, the storage period of a whole blood sample for measuring a Th7R·CD62L^{low} cell subpopulation can be, for example, 1 day to 1 week, 1 day to 6 days, 1 day to 3 days, etc.

Meanwhile, for CCR7 in CD4⁺ cells, variation from the measurement results for a sample immediately after blood collection would be significant after two days from blood collection. Thus, it was found that storage of a whole blood sample for 3 or more days was not suitable for measuring CCR7 in CD4⁺ cells. Measurement results were stable at a storage temperature of 18°C to 25°C up to two days.

For CD8⁺ cells, two days were the limit for stable fluorescence intensity of CD8. A temperature of 18-25°C was suitable for a short period within two days.

In this manner, it was found that the storage temperature or storable period of a whole blood sample varies depending on the type of cells or type of marker to be measured, but a sample can be stored at an expectedly broad temperature range (at about 10°C to about 40°C, more preferably at about 12°C to about 25°C) for a long period of time (within one week, preferably within 6 days, more preferably 3 days from blood collection, such as 1 day to 1 week, 1 day to 6 days, or 1 day to 3 days) for Th7R.

While a whole blood sample can be collected at an amount commonly used in the art, the amount is typically about 16 ml or less, etc. However, as shown in Example 6 herein, Th7R was able to be stably measured, even at a minute amount of about 100 µl. Such an amount can be collected by the subjects themselves and is advantageous in view of the low burden on the subjects. For this reason, the amount of a whole blood sample, in a preferred embodiment of the invention, can be about 10 µl or more, about 50 µl or more, more preferably about 100 µl or more, and about 500 µl or less, more preferably about 200 µl or less, and preferably about 100 µl to 150 µl. Examples of kits that can be used for collecting a whole blood sample in the invention include, but are not limited to, MBS capillary (heparin lithium, 100-150 µl collection line) (Micro Blood Science Inc.), blood collection microtube heparin lithium (without separator), 250 µl "Micro Health Tube" (Health Wave Japan Inc.), Capiject II (Terumo), BD Microtainer^{®} blood collection tube and BD Microgard^{™} Closure, etc.

Conventionally, Th7R has been identified by a medical practitioner collecting blood, immediately separating and optionally cryofreezing PBMCs, and transporting the PBMCs to a venue where a measurement is taken. In such a case, a safety cabinet, a cooling centrifuge, and a deep freezer were required at the venue of blood collection, and a safety cabinet, a cooling centrifuge, a CO₂ incubator, a deep freezer, a liquid nitrogen tank, and other specialized facilities were required at the venue of measurement. Even if measurements were to be taken at the venue of blood collection, a safety cabinet and a cooling centrifuge were required. If the remainder of a sample used in a measurement were to be stored, a deep freezer and a liquid nitrogen tank were also required. However, if a whole blood sample can be used as in the present invention, a microcentrifuge alone is sufficient for a measurement at a venue of blood collection. If a whole blood sample is used and the sample is subsequently transported to a measurement facility, a device is not needed at the venue of blood collection. This is a significant advantage compared to conventional methods.

As shown in a Comparative Example, when a stabilizer was added to anticoagulant-treated blood to immobilize cells, detection of CD4 and CXCR3 was problematic. Thus, the method of efficiently measuring Th7R found in a whole blood sample herein was unexpectedly advantageous.

In a preferred embodiment, a whole blood sample is not subjected to one or both of hemolysis and centrifugation before measurement with a measuring instrument such as a flow cytometer. Seepage of data for abundantly present erythrocytes can be problematic for analysis of leukocytes (T cells in the present invention) in a whole blood sample. In many cases, hemolysis or centrifugation is used for removing erythrocytes, but such a process is cumbersome and may result in damage, loss, or aggregation of cells. The present invention may use a simple method of measuring blood without hemolysis or washing of blood (No-Wash, No-Lyse method). In a No-Wash, No-Lyse method, whole blood (about 5-10 µl) is dispensed, reacted with antibodies, diluted with a buffer, and then subjected to measurements. For the measurement, leukocytes may be separated by using a violet laser or by trigger straining.

For instance, leukocytes can be separated from a whole blood sample by simultaneously using side scatter (SSC) of blue laser and violet laser. Leukocytes and erythrocytes have different scatter properties in violet laser. Erythrocytes would have lower light intensity compared to leukocytes due to absorbing light of violet laser.

Alternatively, leukocytes can be separated from a whole blood sample by staining that distinguishes leukocytes from other cells. Erythrocytes, etc. can be gated out by using a CD45 antibody which is a leukocyte marker or a marker specific to each cell group. Since erythrocytes and platelets do not have a nucleus, only the leukocytes can be specifically stained by using a cell permeable dye that can specifically stain a nucleus while viable (Invitrogen^{™}, Vybrant^{™}, DyeCycle^{™} dye, etc.)

### (PBMC)

When an extended period (e.g., more than one week) is required from blood collection to fractionation/measurement of cells upon measuring a cell subpopulation such as Th7R, PBMCs can be isolated, frozen, thawed upon fractionation/measurement of the cells, optionally cultured, and used. In this regard, as disclosed in Example 5 herein, separation of PBMCs after time has passed since the collection of whole blood is not preferable for measurement of Th7R. Thus, it is preferable to isolate and cryopreserve PBMCs within three hours, more preferably within two hours, after blood collection when measuring a cell marker of Th7R, etc. from isolated PBMCs in the present invention.

PBMCs can be isolated and cryopreserved by a method known in the art. Mononuclear cells can be separated by using, for example, a Vacutainer^{™} blood collection tube and cryopreserved by using liquid nitrogen (see Example 1).

### (Fractionation/Separation of Cells)

To measure the cell count of each cell subpopulation such as a CXCR3⁺CCR4⁻CCR6⁺ T cell subpopulation and a CXCR3⁻ CCR4⁻CCR6⁺ T cell subpopulation, the cell count may be found by experimentally removing cells other than the subpopulations of each kind from all cells. For example, cells corresponding to a T cell subpopulation of interest can be separated from peripheral blood without using a given antibody by using a CD4⁺ Effector Memory T cell isolation kit, human (Militenyi Biotech), etc. For example, cells corresponding to a CD4⁺CD62L^{low} T cell subpopulation can be separated from peripheral blood without using a CD4 antibody and CD62L antibody when a CD4⁺ Effector Memory T cell isolation kit, human (Militenyi Biotech) is used. The total viable cell count can be counted and recorded, and the number of cells obtained using this kit can be counted and recorded. As a similar kit, CD4⁺CD25⁺CD127dim/- Regulatory T cell isolation kit, human (Militenyi Biotech) or CD25⁺CD49d⁻ Regulatory T cell isolation kit, human (Militenyi Biotech) can also be selected. In one embodiment of the invention, any kit that can measure the cell count of each T cell subpopulation of interest can be used.

An antibody does not need to be used. Antibodies that can specifically recognize and bind a molecule expressed on individual cells are prepared so that they can emit color when bound to a molecule expressed on the cell surface or inside the cells. The antibodies are then detected to measure the number of cells that are emitting color. Since the molecules expressed on the cell surface or inside the cells are proteins, mRNA encoding a protein when the protein is expressed is also formed in the cells. In other words, it is sufficient to examine mRNA in individual cells to examine the presence/absence of mRNA encoding a protein of interest. This is made possible by a single cell gene expression analysis, i.e., mRNA analysis at a single cell level. Examples of single cell gene expression analysis include 1) a method of performing next generation sequencing using Quartz-Seq, 2) a method of isolating cells using a Fluidigm C1 System or ICELL8 Single-Cell System to prepare a library with SMART-Seq v4, 3) a method of separating cells with a cell sorter and measuring the cells with quantitative PCR using an Ambion Single Cell-to-CT kit, 4) CyTOF SYSTEM (Standard Biotools), etc.

Specifically, blood is obtained, viable cells are counted, and cells are separated with a cell sorter, etc. For example, Ambion Single Cell-to-CT kit can be used on the individual separated cells to measure the expression level of a specific gene with an apparatus for quantitative PCR. Based on the result, individual cells can be examined as to which subpopulation such as the CXCR3⁺CCR4⁻CCR6⁺ or CXCR3⁻ CCR4⁻CCR6⁺ T cell subpopulation the cells fall under to count the number of cells falling under each subpopulation.

Measurement of the ratio of cell subpopulations or comparison with a reference value or threshold value in the present invention may use a standard sample with a defined signal. Signals can be compared between a standard (e.g., particle to which a fluorescent pigment is attached) prepared to produce a fluorescent signal corresponding to a given cell subpopulation and a sample comprising a cell population to measure the amount or ratio of a cell subpopulation in the sample by comparison with the standard. Signals can also be compared between a standard (e.g., particle to which a fluorescent pigment is attached) prepared to produce a fluorescent signal corresponding to a predetermined reference value or threshold value and a sample comprising a cell population to determine the presence/absence or the amount of the marker of the invention in the T cell composition in the sample by comparison with the standard.

When determining a specific marker to be high (high expression) or low (low expression) in the present invention, those skilled in the art can use a reference classification for expression intensity that is commonly used in the art. For example, it is possible to clearly divide CD62L into CD62L^{low} and CD62L^{high} using the signal intensity corresponding to a 10E2 signal when using a PE-labeled anti-human CD62L antibody as the boundary (WO 2018/147291).

In one embodiment of the invention, those skilled in the art can suitably identify a surface marker of the shown cells to fractionate or count the cells.

As used herein, "flow cytometry" refers to a technology of measuring the number of cells, individuals, and other biological particles suspended in a liquid and individual physical/chemical/biological attributes. Various cells are analyzed using the flow cytometry technology. In particular, differentiation of blood cells can be determined using the flow cytometry technology. Such a determination of differentiation is starting to be used in diagnosis in addition to research. Examples of advantages of flow cytometry include: the ratio accounted for by blast cells can be readily found; specificity and sensitivity are high; it is highly reproducible; a large number of cells can be analyzed; the time required is short; etc.

An apparatus using this technology is referred to as a "flow cytometer". A flow cytometer is an equipment for measuring optical characteristics of a suspended matter (cell) from a homogeneous suspension of cells. Cells pass through the focal point of a laser beam on a liquid flow. When passing through, optical characteristics of forward scatter, side scatter, and one or more fluorescent light with different wavelengths can be simultaneously measured for individual cells at 500 to 4000 cells per second to quickly and accurately measure biological characteristics such as the cell size, internal structure, amounts of various antigens or nucleic acids that are present in the cell membrane, cytoplasm, or nucleus.

Scattered light is light scattered to the surroundings after a laser hits a cell. Forward scatter (FSC) is detected in front with respect to the laser optical axis. The scatter light intensity is proportional to the surface area of a cell. In other words, it is understood that a cell is large if the FSC value is relatively large, and the cell is small if the FSC value is small. Side scatter (SSC) is detected at a position that is 90° (perpendicular) to the laser optical axis. The scattered light intensity is proportional to the state of intracellular structure or cell granule. In other words, it is understood that the internal structure of a cell is complex if the SSC value is relatively large, and the internal structure of a cell is simple if the SSC value is small.

Results of flow cytometry can be typically expressed as a dot plot, with FSC on the X axis and SSC on the Y axis. Each cell is represented by a single dot (point) in the graph. The position thereof is determined by the relative values of FSC and SSC. Lymphocytes with a relatively small size and simple internal structure are displayed in the bottom left section, granulocytes with a large size and a granule inside are displayed in the top right section, and mononuclear cells with a large size but a simple internal structure are displayed between the lymphocytes and the granulocytes, with each forming a population separated from one another.

Fluorescent light refers to a light generated when a fluorescent pigment labeling a cell is excited by an irradiated laser beam and releases energy. A flow cytometer (e.g., product name: Becton & Dickinson FACSCalibur) typically irradiates a 488 nm single wavelength laser beam and a 635 nm single wavelength laser beam. A cell itself also has a property of emitting weak fluorescence (autofluorescence). However, when actually attempting to specifically detect a molecule of a cell using fluorescence, it is necessary to bind a fluorescent pigment to a cell or its molecule in some form in advance. For example, FITC (Fluorescein isothiocyanate) absorbs a 488 nm excitation light and mainly emits a 530 nm fluorescent light (green). Labeling of an antibody with FITC in advance leads to a difference in the amount of antibodies bound in accordance with the amount of antigens on the cell surface, resulting in a difference in the fluorescence intensity of FITC. Thus, the amount of antigens that are present on the cell surface can be estimated. FACSCalibur that can be used as an example has four fluorescence detectors installed, which can detect different fluorescence wavelength regions. If multiple fluorescent pigments that emit light of different wavelengths are prepared, up to four different antigens can be simultaneously detected. As a fluorescent pigment other than FITC which is excited by a 488 nm single wavelength laser beam, PE (phycoerythrin) mainly emits a 585 nm fluorescent light, and PerCP (peridinin chlorophyll protein) and PE-Cy5 (carbocyanin-5) mainly emit a 670 nm fluorescent light. APC (allophycocyanin), which is a fluorescent pigment excited by a 635 nm single wavelength laser beam, mainly emits a 670 nm fluorescent light. These fluorescent pigments are combined with various antibodies and used in double or triple staining of cells. CD4, CD8, CD62L, CD25, CCR4, CCR6, and CXCR3 molecules, etc. that are expressed on the surface of T lymphocytes can be detected with a monoclonal antibody, which specifically reacts thereto.

In the method of the invention, the manner of expression of a cell marker can be determined by any method for analyzing the distribution of the expression of marker proteins such as flow cytometry, gene analysis, or CyTOF. Typically, the manner of expression of a cell marker can be determined in the method of the invention on a cell distribution diagram showing results of a flow cytometer (also referred to as flow cytometry) such as a histogram or dot plot. In particular, a boundary is set (gated) between a CCR4⁻ cell subpopulation and a CCR4⁺ cell subpopulation in a cell distribution diagram of a CXCR3⁻ cell subpopulation and a relative amount of a CCR4⁻CCR6⁺ cell subpopulation in a CD4⁺ T cell population is measured by using the boundary (gate) .

Strictly speaking, there are two types of flow cytometers, i.e., equipment which only analyzes cells and equipment capable of sorting analyzed cells. The latter is known as "FACS". As used herein, "FACS" is an abbreviation of fluorescence-activated cell sorter and refers to an apparatus used in a method of analyzing a surface antigen of a free cell such as a lymphocyte using a laser beam or sorting a specific cell by the presence/absence of a surface antigen, etc.

The results of flow cytometry can be displayed by distributing cells as a distribution chart of light derived from particles, such as a histogram or dot plot (scatter plot), for a single measured item or for each of two or more measured items, from the detected optical information. As used herein, "histogram" refers to a graph representing intensity of an optical signal of each parameter on the X axis and the cell count on the Y axis in fluorescence measurement using a flow cytometer. 10 thousand or more cells can be counted in total in such a form. As used herein, "dot plot" refers to a plot of fluorescence intensity of two types of fluorescent pigments on the X and Y axes. When double- or triple-stained, this can be analyzed using a display method in which the respective fluorescence intensity is placed on the X or Y axis and individual cells correspond to each point on a two-dimensional graph. As used herein, "gating", "setting a boundary", or "providing a gate" refers to selecting a certain distribution region in accordance with a measured item in a distribution chart in order to take a proper measurement in accordance with the measured item for each distribution chart.

For example, peripheral blood or bone marrow fluid can be collected, and then erythrocytes can be removed by a hemolytic method or specific gravity centrifugation, then the residual can be reacted with a fluorescently labeled antibody (antibody to antigen of interest and a control antibody thereof) and sufficiently washed for observation using flow cytometry. The detected scattered light or fluorescence is converted to an electric signal and analyzed by a computer. The result can distinguish lymphocytes, mononuclear cells, and granulocytes by representing the intensity of FSC as the cell size and the intensity of SSC as intracellular structure. The cell population of interest is gated thereafter as needed to examine the manner of expressing a cell marker in the cells.

In practicing the method of the invention, those skilled in the art can suitably identify a surface marker of the shown cells to fractionate or count the cells.

As discussed with regard to a No-Wash, No-Lyse method, the objective of the present invention is to measure the ratio of a specific cell subpopulation in T cells by targeting whole blood or PBMCs. Thus, data analysis may be facilitated by adding a CD45 antibody. Since CD45 antibodies react with lymphocytes including T cells but not with erythrocytes or platelets, the subject of measurement can be limited to a CD45^{bright} cell subpopulation to exclude unnecessary cells such as erythrocytes and platelets from subjects of analysis.

### (Kit)

In one embodiment of the invention, the kit of the invention can comprise a detecting agent for CD4, CCR4, and CCR6 for use in a sample prepared in accordance with the method of the invention. In another embodiment, the kit of the invention can further comprise a detecting agent for CXCR3 or CD62L. In one embodiment, a detecting agent is an antibody. Preferably, an antibody is suitably labeled and facilitates the detection of a marker.

### (General technology)

The molecular biological methodology, biochemical methodology, and microbiological methodology used herein are well known and conventionally used in the art, which are described, for example, in Sambrook J. et al. (1989). Molecular Cloning: A Laboratory Manual, Cold Spring Harbor and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J. J. et al. (1999). PCR Applications: Protocols for Functional Genomics, Academic Press, Bessatsu Jikken Igaku [Experimental Medicine, Supplemental Volume*],* "Idenshi Donyu & Hatsugen Kaiseki Jikken Ho [Experimental Methods for Transgenesis & Expression Analysis*]",* Yodosha, 1997, etc. Relevant portions thereof (which may be the entire document) are incorporated herein by reference.

As used herein, "or" is used when "at least one or more" of the listed elements in the sentence can be employed. When explicitly described herein as "within the range" of "two values", the range also includes the two values themselves.

Reference literature such as scientific literature, patents, and patent applications cited herein are incorporated herein by reference to the same extent that the entirety of each document is specifically described.

The present invention has been described while showing preferred embodiments to facilitate understanding. While the present invention is described hereinafter based on the Examples, the above descriptions and the following Examples are provided for the sole purpose of exemplification, not limitation of the present invention. Thus, the scope of the present invention is not limited to the embodiments and Examples that are specifically described herein and is limited only by the scope of claims.

### [Examples]

### (Measurement panel)

In the Examples, the following panel in Table 1 (WB panel 2 or WB panel 3) was used.

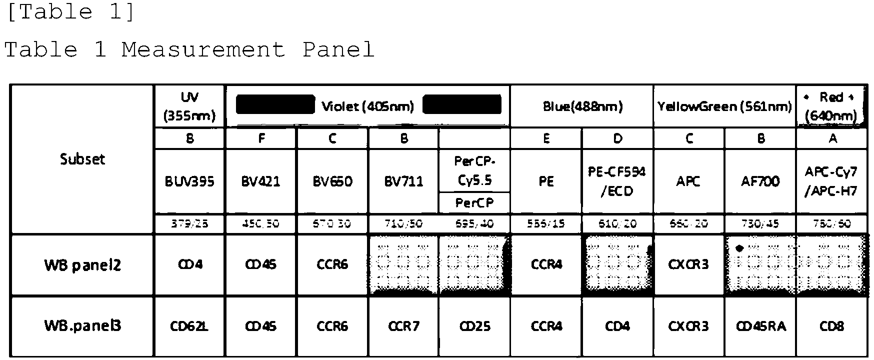

### (Example 1: Comparison of whole blood method and method of separating PBMCs)

Whole blood was collected in an anticoagulant (heparin sodium)-containing blood collection tube and compared with an embodiment where PBMCs were immediately separated from whole blood and cryopreserved. The samples analyzed were 8 ml of blood collected from the same subject (H-34), which were treated with heparin. This was separated into two, and one of them was used as heparin treated whole blood and the other was used for separating PBMCs.

For the whole blood, a staining antibody was added to 100 µl of the whole blood, which was kept away from light for 15 minutes and stained at room temperature. 2 ml of hemolytic reagent (lysing solution (Becton Dickinson, Cat. 349202) was then added, and the blood was kept away from light for 10 minutes and left at room temperature to lyse erythrocytes. Then, after centrifugation at 500×g at room temperature for 5 minutes, the supernatant was discarded. Furthermore, 2 ml of 2% FBS/PBS was added to suspend the cells, which were centrifuged under the same conditions. The suspension and centrifugation were repeated again. After removing the supernatant, the cells were suspended in 250 µl of 2% FBS/PBS to take measurements.

On the other hand, to obtain PBMCs, the remaining whole blood was transferred to a 15 ml conical tube and mixed after adding saline at an amount that is two-fold amount of the amount of blood. The mixture was dispensed into two Leucosep lymphocyte separation tubes (Greiner, Cat. 163288-013) and centrifuged at 800×g at room temperature for 15 minutes. The top layer consisting of plasma and cells was stirred by pipetting, and an amount corresponding to two Leucosep lymphocyte separation tubes was collected in one 50 ml conical tube. 15 ml of 5% FBS/PBS was added to the collected cellular solution, and the mixture was centrifuged at 500×g at 4°C for 5 minutes. After removing the supernatant, 10 ml of 5% FBS/PBS was added to suspend the cells. The cells were counted, and the cellular solution was divided into a solution for measurement on the same day and a solution for freezing.

The solution for measurement on the same day was centrifuged after adding 10 ml of 5% FBS/PBS again; after removing the supernatant, 50 µl of PBS was added to suspend the cells; the suspension was transferred to a FACS tube, and a staining antibody was immediately added to the suspension, which was kept away from light for 20 minutes and stained at room temperature; subsequently, 1 ml of 2% FBS/PBS was added to suspend the cells, and the suspension was centrifuged at 500×g at 4°C for 5 minutes; after removing the supernatant, 250 µl of 2% FBS/PBS was added to suspend the cells; and measurements were taken (which is the PBMC straining step). The above was deemed as Fresh PBMC.

The solution for freezing was centrifuged at 500×g at 4°C for 5 minutes. After removing the supernatant, 1 ml of Cellbanker 2 was added to suspend the cells. The entire amount thereof was dispensed into a cryotube, placed in a BICELL cooled to 4°C, then quickly placed in a -80°C deep freezer. On the next day, the cells were transferred to and cryopreserved in a liquid nitrogen tank. After 5 days, the cells were melted in a thermostatic vessel held at 37°C, then suspended in 12 ml of 10% FBS/RPMI 1640 (Nacalai Tesque, Cat. 30264-85) and centrifuged at 500×g at 4°C for 5 minutes, and the supernatant was removed. The cells were suspended again by adding 10 ml of 10% FBS/RPMI 1640 medium. The cells were counted, then the cellular solution was divided into two. One part of the cellular solution was centrifuged at 500×g at 4°C for 5 minutes, suspended by adding 100 µl of PBS after removing the supernatant, and subjected to the PBMC staining step to take measurements. The cellular solution was deemed as Frozen. The other cellular solution was dispensed in a 24-well plate and cultured at 37°C in the presence of 5% CO₂ for 48 hours. Subsequently, the entire amount of the culture solution comprising the cells was collected in a 15 ml conical tube, and PBS was added to increase the volume to 10 ml, then the cells were counted. The cellular solution was then centrifuged at 500×g at 4°C for 5 minutes, suspended by adding 100 µl of PBS after removing the supernatant, and subjected to the PBMC staining step to take measurements. The cellular solution was deemed as PBMC Culture. The same applies hereinafter.

CD45, CD4, CCR4, CCR6, and CXCR3 antibodies were used for staining (Table 1: WB panel 2), and BD Fortessa X-20 was used to take measurements. The measurement results were analyzed with FlowJo^{™} (Becton Dickinson) and diagrams were drawn.

Diagrams for whole blood and Fresh PBMCs are similar (Figure 1). Specifically, in order from the top row, each of the patterns depicting a scatter plot of CCR4 vs CCR6 for the entire CD4⁺ cells, those depicting a histogram of CXCR3 for CD4⁺ cells, those depicting a scatter plot of CCR4 vs CCR6 by gathering CXCR3⁻ in the histogram, and those depicting a scatter plot of CCR4 vs CCR6 by gathering CXCR3⁺ are similar to the patterns for whole blood and Fresh. Meanwhile, Frozen PMBCs are different from whole blood and Fresh PBMCs in each diagram. For Culture PBMCs, CCR6 exhibits an intermediate pattern of Fresh and Frozen PBMCs, but CCR4⁺ is reduced relative to Fresh and Frozen PBMCs in the three (CD4⁺, CXCR3⁻, and CXCR3⁺) scatter plots of CCR4 vs CCR6. The histogram of CXCR3 is close to that of Frozen PBMCs.

The following were elucidated in view of the above.
*Whole blood is similar to Fresh PBMCs.
*PBMCs, when cryopreserved, are different from whole blood and Fresh PBMCs, and are different from Fresh PBMCs even after culture.
*Separation of CXCR3 deteriorates after cryopreservation and does not recover even after culture.
*The CCR4⁺ count does not decrease by cryopreservation but decreases after culture.
*The CCR6⁺ count decreases after cryopreservation and recovers somewhat after culture but is less than that immediately after blood collection.

### (Example 2: Study of effect of storage period)

This Example took measurements by changing the storage period of whole blood to study the effect of storage period.

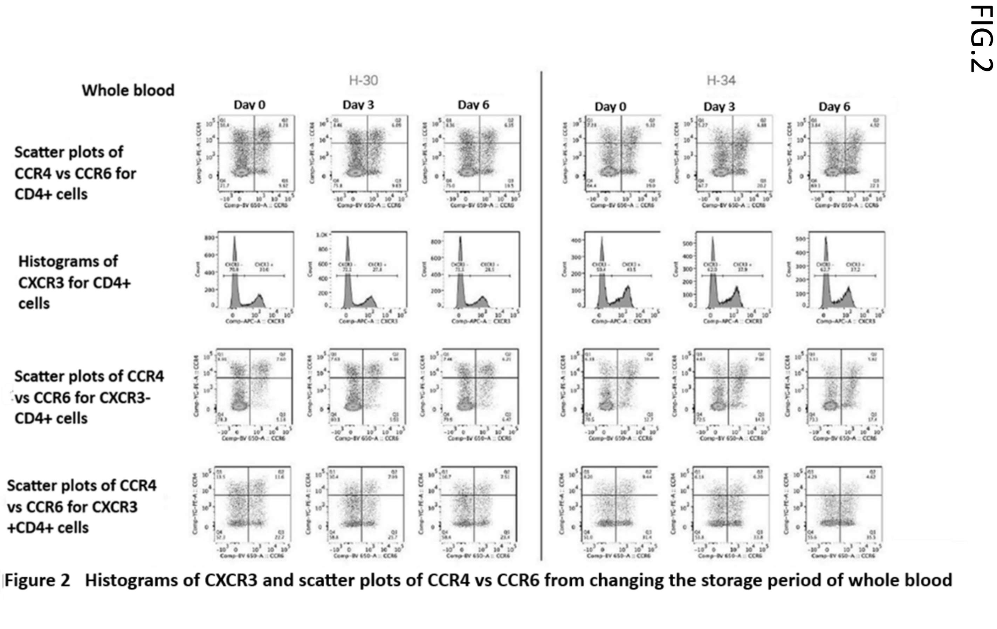

Whole blood immediately after blood collection, whole blood stored for three days at room temperature (20-22°C), and whole blood stored for six days at room temperature were prepared. Whole blood was lysed, stained, and measured, immediately or after storage. An experiment was conducted by using each of a whole blood sample (H-30) prepared in Example 1 and a whole blood sample (H-34) collected on a different day. WB panel 2 was used as the measurement panel (Table 1). A significant difference was not found between results immediately after blood collection and those after storage (Figure 2).

Furthermore, the number of samples was increased, Th7R cells were counted, and the number of days of storage of whole blood was compared.

The whole blood sample (H-30) prepared in Example 1 and the whole blood sample (H-34) collected on a different day were continued to be used as whole blood. The results are shown in Table 2. H-34 in the table shows the results in Figure 2 as numerical values.

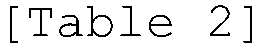

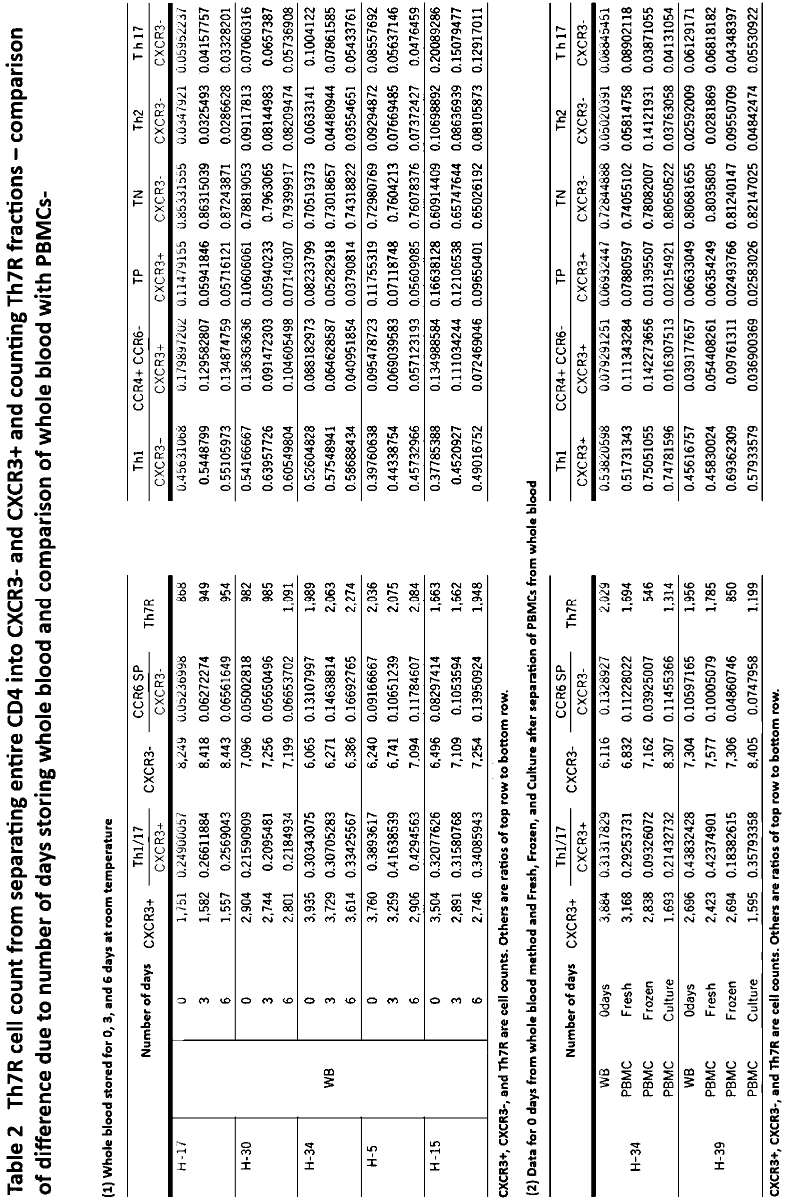

Table 2 also compares whole blood to PBMCs (Table 2, bottom row, "2. Data for 0 days from whole blood method and Fresh, Frozen, and Culture after separation of PBMCs from whole blood"). The method of treating PBMCs was discussed in Example 1. H-34 in the bottom row of Table 2 shows the results of Example 1 (Figure 1) as numerical values. WB panel 2 was used as the measurement panel. Histograms and scatter plots corresponding to Figure 1 were created for each sample. FlowJo was used to draw the diagrams, and the cell count was obtained. The cell counts for CXCR3⁻ and CXCR3⁺ were obtained from the histogram of CXCR3.

In the top row of Table 2 (1. Whole blood stored for 0, 3, and 6 days at room temperature), 10,000 or more CD4⁺ as a whole were obtained, and CD4⁺ as a whole was adjusted to be 10,000 thereafter upon creating the table.

The bottom row of Table 2 (2. Data for 0 days from whole blood method and Fresh, Frozen, and Culture after separation of PBMCs from whole blood) shows the number calculated after obtaining about 10,000 CD4⁺ as a whole by FlowJo.

Th1/17 is the cell count in the region of CCR4⁻CCR6⁺ in the scatter plot of CCR4 vs CCR6 for CXCR3⁺. The ratio relative to CXCR3⁺ is shown.

CCR6 SP (Single positive) is the cell count in the region of CCR4⁻CCR6⁺ in the scatter plot of CCR4 vs CCR6 for CXCR3⁻. The ratio relative to CXCR3⁻ is shown.

Th7R indicates the total number of Th1/17 and CCR6 SP.

Th1 is the cell in the region of CCR4⁻CCR6⁻ in the scatter plot of CCR4 vs CCR6 for CXCR3⁺. The ratio relative to CXCR3⁺ is shown.

CCR4⁺CCR6⁻ is the cell in the region of CCR4⁺CCR6⁻ in the scatter plot of CCR4 vs CCR6 for CXCR3⁺. The ratio relative to CXCR3⁺ is shown.

TP (Triple positive) is the cell in the region of CCR4⁺CCR6⁺ in the scatter plot of CCR4 vs CCR6 for CXCR3⁺. The ratio relative to CXCR3⁺ is shown.

TN (Triple negative) is the cell in the region of CCR4⁻ CCR6⁻ in the scatter plot of CCR4 vs CCR6 for CXCR3⁻. The ratio relative to CXCR3⁻ is shown.

Th2 is the cell in the region of CCR4⁺CCR6⁻ in the scatter plot of CCR4 vs CCR6 for CXCR3⁻. The ratio relative to CXCR3⁻ is shown.

Th17 is the cell in the region of CCR4⁺CCR6⁺ in the scatter plot of CCR4 vs CCR6 for CXCR3⁻. The ratio relative to CXCR3⁻ is shown.

### (Discussion)

### 1. Difference due to number of days storing whole blood

When whole blood measured on the day of blood collection, whole blood measured after storage for 3 days at room temperature, and whole blood measured after storage for 6 days at room temperature were compared, there was a tendency of CXCR3⁺ decreasing but the ratio of Th1/17 in CXCR3⁺ slightly increasing, as the storage period increased (Table 2). CXCR3⁻ increased and the ratio of CCR6 SP in CXCR3⁻ also increased. Further, Th7R, which is the sum of Th1/17 and CCR6 SP, increased somewhat. Specifically, CXCR3⁺ decreased, CXCR3⁻ increased, and Th7R which is the total of CCR4⁻CCR6⁺ increased. However, as can be understood from Figure 2, the increase/decrease was small in each case. As discussed below, this is close to the data for Fresh PBMCs compared to Frozen or Culture PBMCs. In other words, it can be understood that the change during storage of whole blood is small compared to other treatment methods.

Th1 accounting for CXCR3⁺ and TN accounting for CXCR3⁻, i.e., the ratio of CCR4⁻CCR6⁻, increased slightly. Thus, the ratio of CCR4⁻CCR6⁻ increased.

The ratio of CCR4⁺CCR6⁻ in CXCR3⁺ and the ratio of Th2 in CXCR3⁻ decreased, i.e., the ratio of CCR4⁺CCR6⁻ decreased.

Both the ratio of TP (CCR4⁺CCR6⁺) in CXCR3⁺ and the ratio of Th17 in CXCR3⁻ decreased.

If the ratio in CCR4 vs CCR6 is reviewed together with the previous results for Th1/17 and CCR6 SP, the tendency of increase/decrease in the ratio matched, regardless of whether CXCR3 is - or +. CCR4⁻CCR6⁻ and CCR4⁻CCR6⁺ increased, and CCR4⁺CCR6⁻ and CCR4⁺CCR6⁺ decreased. In other words, CCR4⁺ decreased and CCR4⁻ increased. However, the difference was not to the extent of that relative to PBMCs.

### 2. Comparison of whole blood with PBMCs

Whole blood had more CXCR3⁺ and less CXCR3⁻ compared to Fresh PBMCs (Table 2). Th1/17 and CCR6 SP as well as Th7R were also slightly higher, but the difference was small.

As for the ratio of cells in a region separated in CCR4 vs CCR6, whole blood resulted in having about the same Th1, TP, and TN, less CXCR3+CCR4+CCR6-, slightly less Th2, and slightly less Th17, relative to Fresh PBMCs. However, the difference was small relative to the comparison with Frozen or Culture PBMCs.

Comparison is now made while including Frozen and Culture PBMCs. CXCR3⁺ varies depending on the sample in Frozen PBMCs relative to Fresh but is significantly less in Culture. On the other hand, CXCR3⁻ in Frozen is close to that in Fresh and it is higher in Culture than Fresh. The ratio of Th1/17 with respect to CXCR3+ is significantly less in Frozen PBMCs than Fresh, and higher in Culture than Frozen but less than Fresh. The ratio of CCR6 SP is similar to Th1/17, i.e., significantly less in Frozen and slightly less in Culture than Fresh. The number of Th7R is significantly less in Frozen than Fresh and higher in Culture than Frozen but less than Fresh.

In other words, the number of CXCR3⁺ decreased significantly after culture, so that the number of Th1/17 decreased significantly after cryopreservation. The number increased slightly after culture but did not recover to the level immediately after separation. Meanwhile, the ratio of CCR6 SP decreased significantly by cryopreservation, but there were some samples where the ratio of CCR6 SP after culture increased to about the level before cryopreservation and some samples where the ratio increased only slightly. When measured by fixing the number of CD4+ cells, the number of CXCR3⁻ increased after culture compared to that immediately after separation, but the number of CCR6 SP increased or decreased depending on the sample. Thus, Th7R, which is the sum of Th1/17 and CCR6 SP, decreased after cryopreservation and increased after culture but did not recover to the level immediately after separation. In other words, when measuring Th7R with PBMCs, it is desirable to take measurements before cryopreservation immediately after separation.

Comparison was made for cells in regions other than CCR4⁻ CCR6⁺ in CCR4 vs CCR6, including Frozen and Culture PBMCs. The ratio of Th1 with respect to CXCR3⁺ slightly increased in Frozen relative to Fresh and was slightly lower in Culture than Frozen but higher than Fresh. The ratio of CCR4⁺CCR6⁻ increased in Frozen and decreased significantly in Culture. The ratio of TP decreased significantly in Frozen and increased in Culture but was much less than Fresh. For CXCR3⁻ TN, Th2, and Th17, the expression patterns of CCR4 and CCR6 had the same tendency as the increase/decrease of Th1, CCR4⁺CCR6⁻, and TP, respectively, corresponding to CXCR3⁺. This also applies to the relationship between Th1/17 and CCR6 SP. In other words, the effect of cryopreservation or culture was not associated with the presence/absence of CXCR3. In summary, CCR4⁺ cells were not affected by cryopreservation but decreased when cultured. CCR6⁺ cells decreased upon cryopreservation but recovered when cultured. However, CCR4⁺CCR6⁺ was affected by a decrease of both CCR4⁺ cells and CCR6⁺ cells, i.e., decreased upon cryopreservation and did not recover after being cultured.

### (Example 3: Effect of storage period (2))

Data in Example 2 were used for comparison with the Th7R cell count.

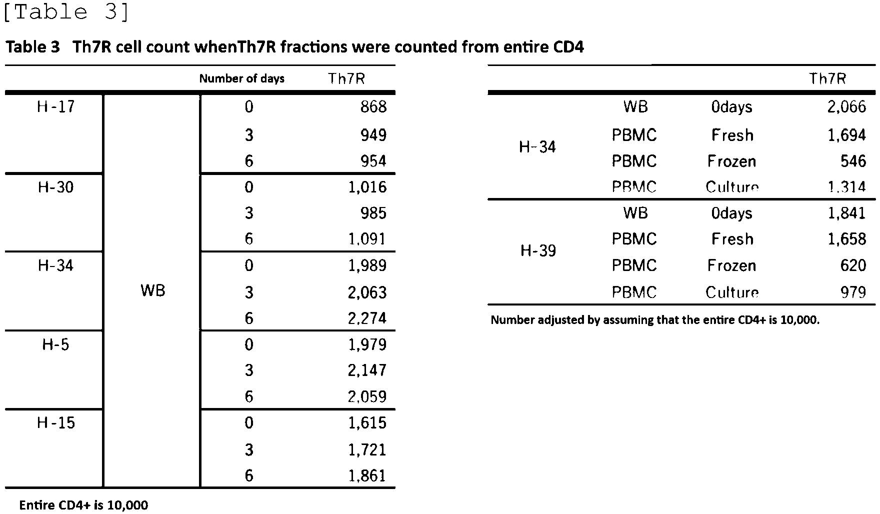

The left side of Table 3 (whole blood) is the same measurement as the top row of Table 2 (1. Whole blood stored for 0, 3, and 6 days at room temperature), and the right side (whole blood and PBMC) is the same measurement as the bottom row of Table 2 "2. Data for 0 days from whole blood method and Fresh, Frozen, and Culture after separation of PBMCs from whole blood".
WB panel 2 (Table 1) was used for the measurement.

In this regard, cells were not separated by CXCR3, and CCR4 vs CCR6 scatter plots were drawn with respect to the entire CD4⁺. The plots are shown in Figures **1** and **2****.** Similar plots were also drawn by FlowJo for those that are not shown in Figures **1** and **2** to count the cells. Since CXCR3⁻/⁺ are not separated, only the Th7R cell counts are shown (Table 3).

A significant difference was not found between whole blood immediately after blood collection and those after storage. In other words, measurements did not have problems even after six days had passed after blood collection.

Th7R was slightly less in Fresh PBMCs than in whole blood.

Th7R decreased more significantly for Frozen PBMCs than whole blood or Fresh. The number in Culture obtained subsequently did not recover to the level of whole blood or Fresh.

In other words, when PBMCs are separated, Th7R needs to be measured immediately after separation. When PBMCs are separated after storage of whole blood, desired measurement results were not obtained (see Example 5). Thus, it is desirable to separate PBMCs immediately after blood collection and take measurements immediately. Meanwhile, the same results as those immediately after blood collection are obtained for whole blood, even after 6 days. Thus, it was revealed that measurements can be taken after transporting blood for 6 days at room temperature.

### (Example 4: Effect of storage temperature (3))

This Example studied the optimal temperature range for storage of whole blood. Tests were conducted twice. The first test took measurements after 1, 2, 3, or 6 days had passed at 10, 15, 20, 25, 30, or 37°C. The second test took measurements after 1, 3, or 6 days had passed at 12, 15, or 18°C. Blood treated with heparin was dispensed into CRYOtubes at 250 µl each and stored in a water bath or incubator set at each temperature. WB panel 2 and WB panel 3 were used for the measurements (Table 1).

In the first test, the possibility was contemplated for water droplets adhering to the inside of the lid to concentrate the blood portion when stored at 15°C. Thus, the second test was conducted by introducing a mechanism for maintaining the temperature so that water droplets would not adhere.

### 1. Expression of CD4 and CD8

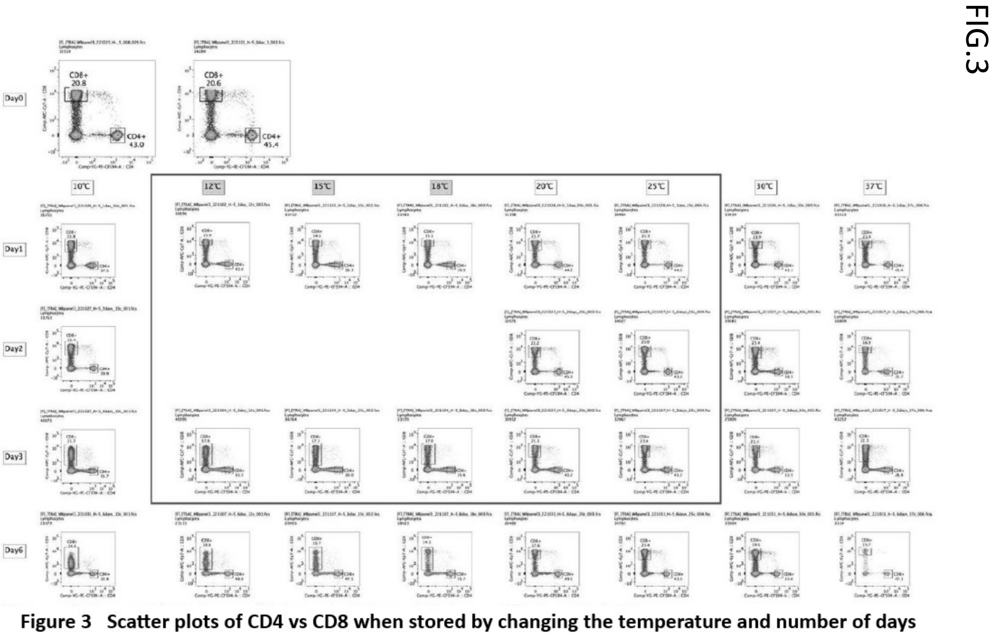

Figure **3** shows scatter plots of CD4 vs CD8 when stored by changing the temperature and number of days. The horizontal axis indicates the distribution of CD4, and the vertical axis indicates the distribution of CD8.

The conditions within the frame of Figure **3** were deemed to be stable. CD4 staining was stable for 3 days between 12°C and 25°C in view of the staining patterns and the frequency of CD4⁺. For CD8, it appears that the fluorescence intensity of CD8 decreased and the expression level decreased after two days at any temperature. In subsequent analysis, the range gated as CD8⁺ was moved in accordance with the decrease in fluorescence intensity.

### 2. Expression of CD62L in CD4

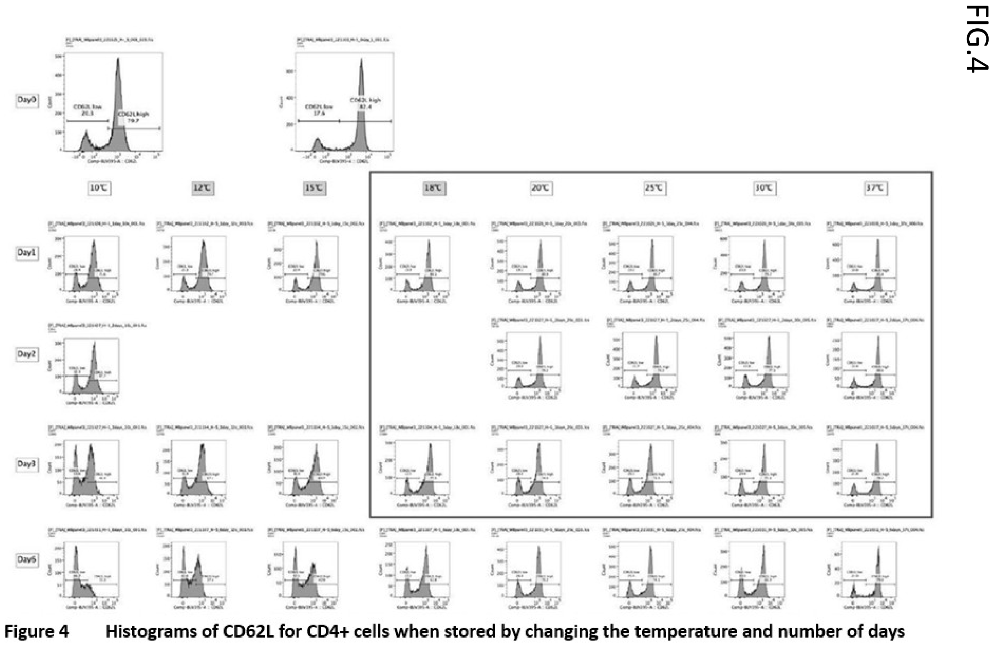

Staining patterns of CD62L for CD4⁺ cells are represented by histograms (Figure **4****).** The horizontal axis indicates the distribution of CD62, and the vertical axis indicates the number counted.

The conditions within the frame of Figure **4** were deemed stable. It was found that better separation, when a number of days has passed, is achieved at higher temperatures for CD62L^{low}. At close to 37°C, staining was stable even if the number of days of storage increased.

### 3. Expression of CD62L in CD8

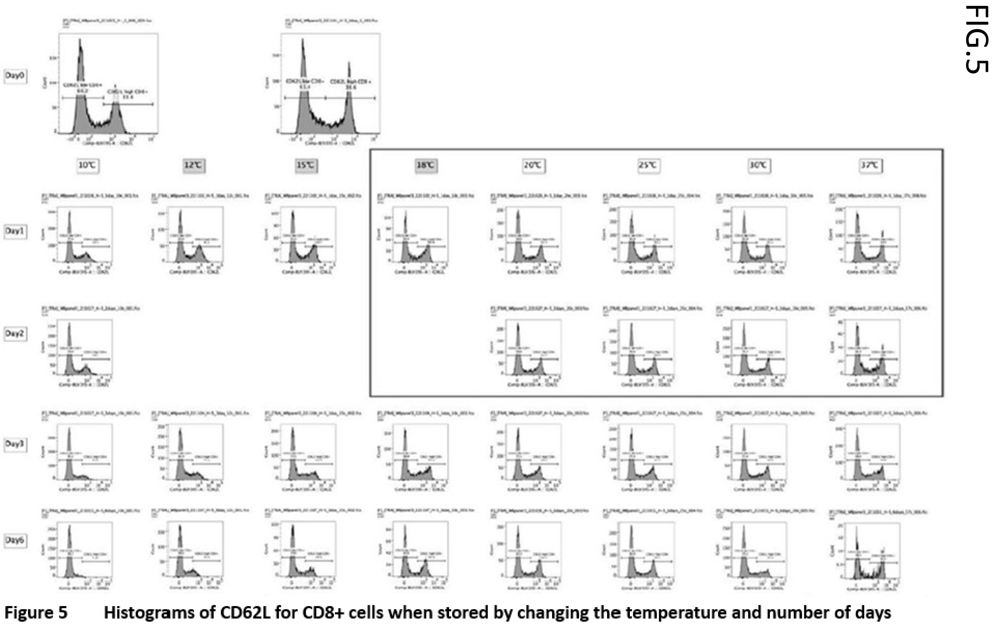

Staining patterns of CD62L for CD8⁺ cells are represented by histograms (Figure **5****).** The horizontal axis indicates the distribution of CD62L, and the vertical axis indicates the number counted.

The conditions within the frame of Figure **5** were deemed stable. CD8⁺ cells were selected, when positions of CD8⁺ decreased, by shifting the range in accordance therewith. At close to 37°C, staining was stable even if the number of days of storage increased, in the same manner as CD4⁺ cells, but the fluorescence intensity as a whole decreased after two days.

### 4. Expression of Th7R in CD4

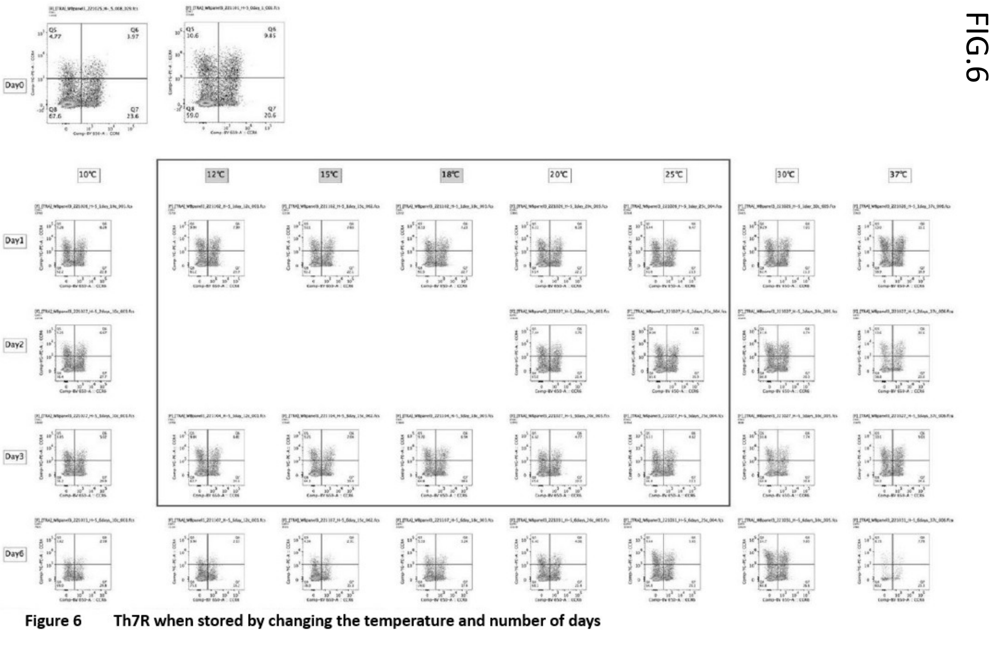

Histograms focusing on cells in a CCR4⁻CCR6⁺ fraction with respect to CD4⁺ cells, i.e., Th7R cells, were drawn (Figure **6****).** The horizontal axis indicates the distribution of CCR6, and the vertical axis indicates the distribution of CCR4.

The conditions within the frame of Figure **6** were deemed stable. Th7R was able to be measured well under storage at a wide range of temperatures at a long storage period including storage for 3 days.

### 5. Expression of CCR7 and CD45RA in CD4

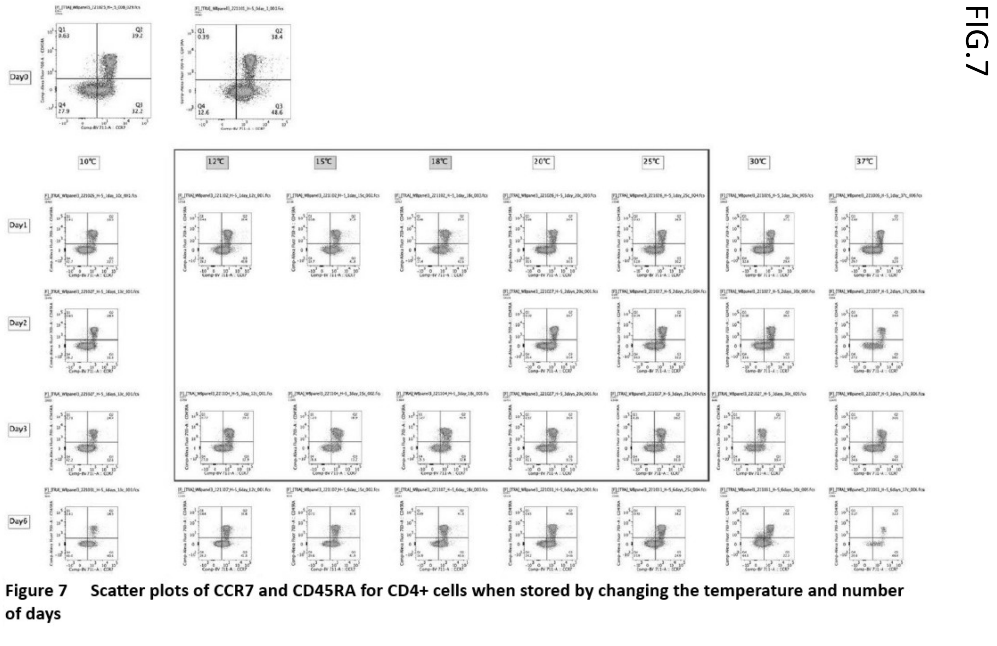

Scatter plots of CCR7 and CD45RA were created for CD4⁺ cells (Figure **7****).** In other words, central memory, effector memory, and naïve cells were observed. The horizontal axis indicates the distribution of CCR7, and the vertical axis indicates the distribution of CD45RA.

The conditions within the frame of Figure **7** were deemed stable. Separation of staining of CCR7 deteriorated as the number of days of storage increased. Thus, effector memory cells (CCR7⁻CD45RA⁻) and effector cells (CCR7⁻CD45RA⁺) increased. For CD45RA, expression tended to decrease at low temperatures, and the frequency was stable near 20°C. Accordingly, for naïve cells (CCR7⁺CD45RA⁺), results were excellent near 20°C. The frequency decreased at lower or higher temperatures.

### 6. Expression of CCR7 and CD45RA in CD8

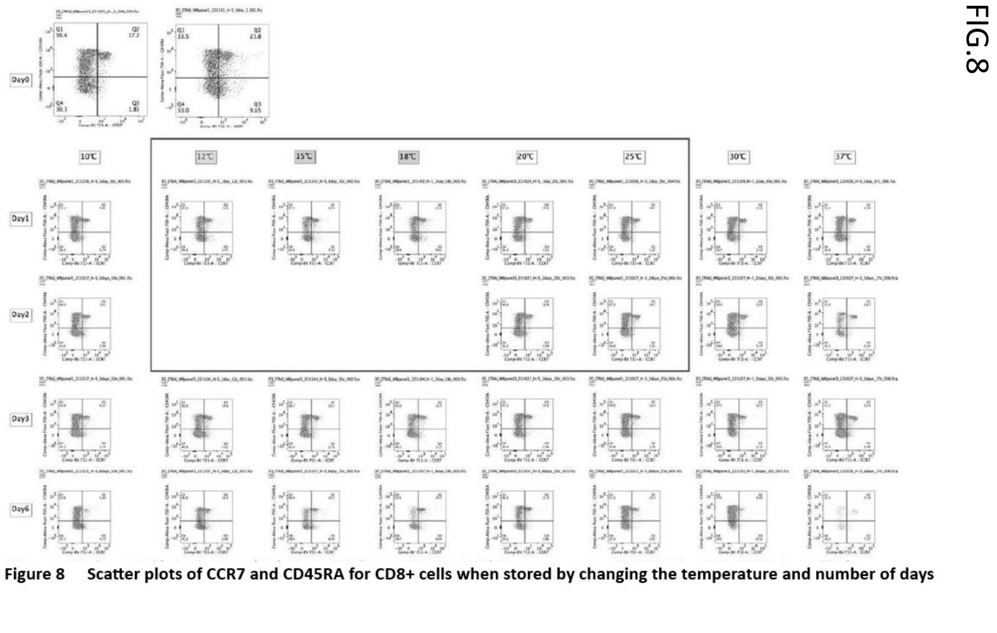

Scatter plots of CCR7 and CD45RA were created for CD8⁺ cells (Figure **8****).** In other words, central memory, effector memory, naïve, and EMRA cells were observed. The horizontal axis indicates the distribution of CCR7, and the vertical axis indicates the distribution of CD45RA.

The conditions within the frame of Figure 8 were deemed stable. Expression of CCR7 and CD45RA was excellent up to two days of storage and was nearly the same as that after one day of storage. After the second day, expression of CCR7 decreased dramatically. Expression of CD45RA also decreased gradually.

### 7. Summary

For measurement of CD8⁺ cells, the fluorescence intensity of CD8 was stable only up to 2 days. A temperature of 18-25°C was suitable for a short period of 2 days or less.

CD4 was stable for 3 days between 12°C and 25°C, but the stability of each marker in CD4⁺ cells varied individually.

Better separation, when a number of days has passed, was achieved at higher temperatures for CD62L. At close to 37°C, stability was exhibited even if the number of days of storage increased.

Separation of staining of CCR7 deteriorated as the number of days of storage increased. Stability was exhibited at 18°C to 25°C as the temperature.

Th7R was able to be measured well under storage at a wide range of temperatures (12°C to 25°C) at a long storage period including storage for 3 days.

### (Example 5: Separation of PBMCs from stored whole blood)

Utility of residual samples after measurement of whole blood was studied. As discussed above, some cell markers cannot be measured due to deterioration if stored as whole blood. In this regard, separation and storage of PBMCs after storage of whole blood was studied.

Blood was collected from two healthy individuals by using heparin blood collection tubes. The following four patterns were prepared:
#1: Measurement immediately after blood collection;
#2: Measurement after storage of #1 for 3 days at room temperature (20.4-21.0°C);
#3: Measurement after separating PBMCs from #2, cryopreservation for 23 days, and thawing the PBMCs; and
#4: Measurement after culturing #3 for 48 hours.
WB panel 2 was used for the measurement (Table 1).

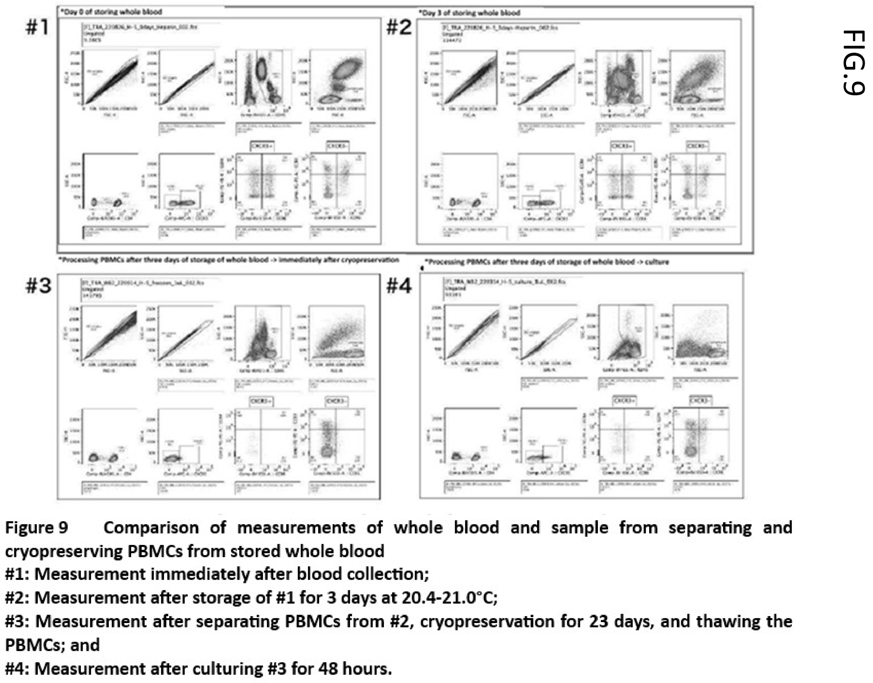

The results are shown in Figure 9.

The following Table 4 shows the frequency of cells obtained from each storage method. Each item in Table 4 indicates the following.
CD45: ratio of CD45 to Singlets
Lymphocytes: ratio of Lymphocytes to CD45
CD4: ratio of CD4 to Lymphocytes
CXCR3⁺: ratio of CXCR3⁺ to CD4
Th1/17: ratio of Th1/17 to CD4
CXCR3⁻: ratio of CXCR3⁻ to CD4
CCR6 SP: ratio of CCR6 SP to CD4

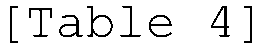

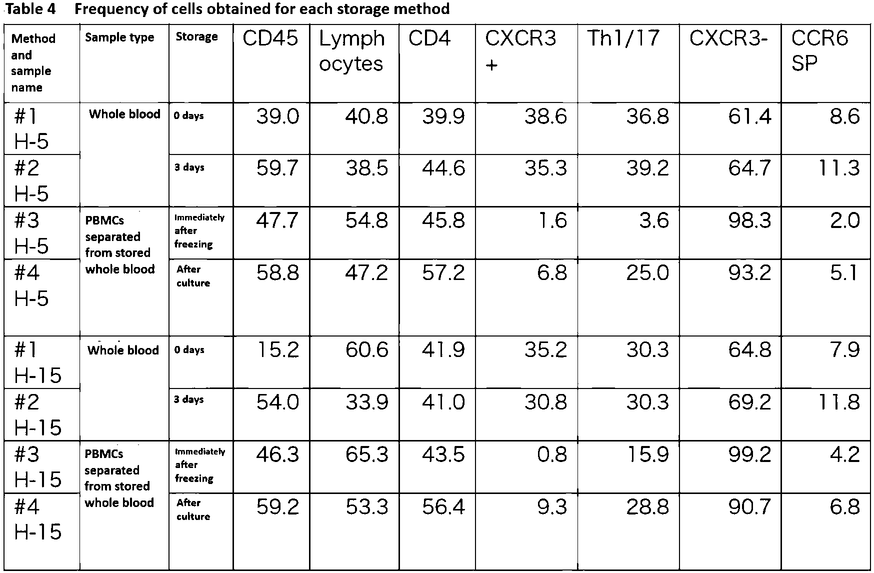

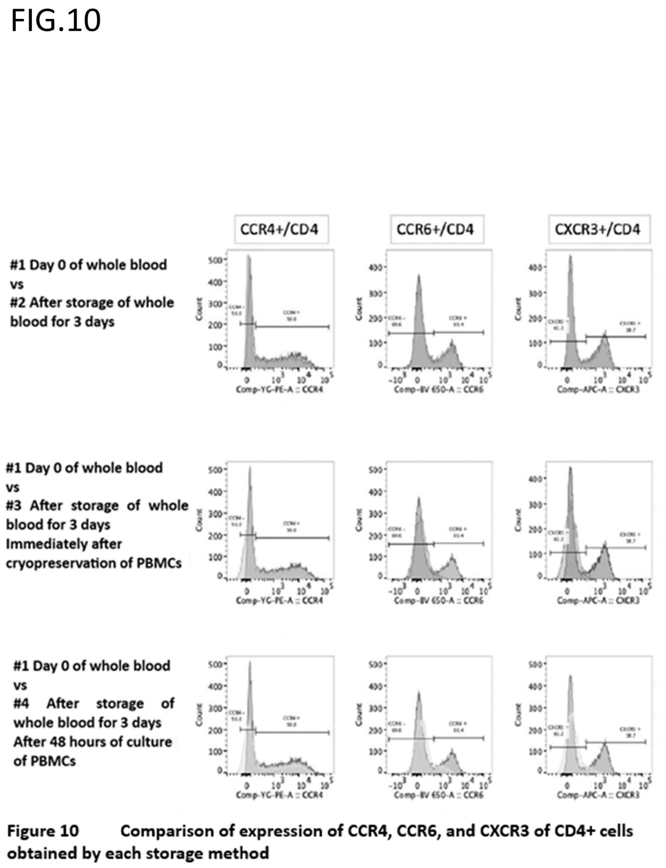

Figure 10 shows the results of comparing expression of CCR4, CCR6, and CXCR3 of CD4⁺ cells for each storage method.

Figure 9 and Figure 10 are for the same samples. Table 4 shows the frequency of cells for two samples. In view of Figure 9 and Table 4, CXCR3⁺ cells were decreased significantly in PBMCs separated from the stored whole blood of #3.
While the cells slightly increased by culture of #4, the number was less than that for whole blood in #1 and #2.

Th1/17, which is CXCR3⁺CCR4⁻CCR6⁺, had the same tendency as CXCR3⁺. However, the ratio after culture was close to that before culture.

CCR6 SP (CXCR3⁻CCR4⁻CCR6⁺) also had the same tendency as CXCR3⁺ despite being CXCR3⁻.

In this regard, Figure **10** is shown to elucidate which marker is affected by the sample treatment conditions. Results obtained immediately after blood collection of #1 were compared to those of #2 to #4. According to the figure, results of #2 Storage of whole blood for three days did not change from #1 for any of the markers. There was no change in CCR4 for #3 and #4 from #1. However, CCR6⁺ and CXCR3⁺ decreased significantly for #3 and #4 relative to #1. CCR6⁺ and CXCR3⁺ slightly increased in #4 compared to #3, but were less than those in #1 or #2.

This shows that separation of PBMCs after three days may not be preferable for measurement of CXCR3 and CCR6 due to the decreased amount of CXCR3⁺ and CCR6⁺ cells. Thus, PBMCs separated after storage of whole blood are not suitable for measurement of Th7R. For storage, it is desirable to separate blood immediately after blood collection into two groups, take measurements within two days for measurement of one group as a whole blood sample including CD8 or within three days for a whole blood sample without measurement of CD8, and separate the remaining blood into PBMCs and cryopreserve the PBMCs immediately after blood collection.

### (Example 6: Importance of measuring CXCR3)

The definition of Th7R is CCR4⁻CCR6⁺CD4⁺, including CXCR3⁺ and CXCR3⁻. Thus, measurement of CXCR3 is thought to be unnecessary for measurement of Th7R, but this Example studied whether there is significance in measuring CXCR3.

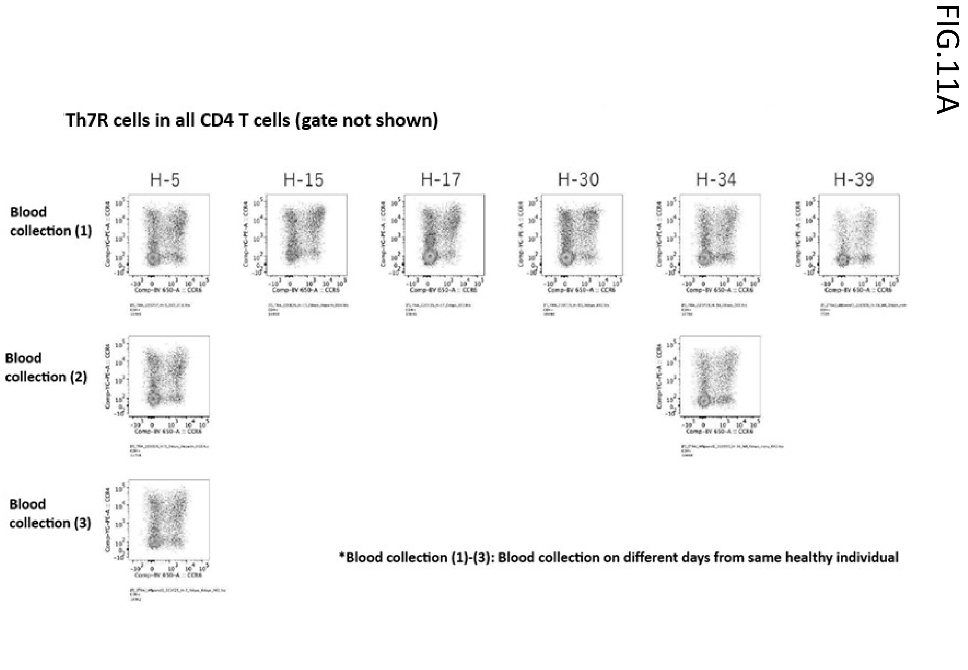

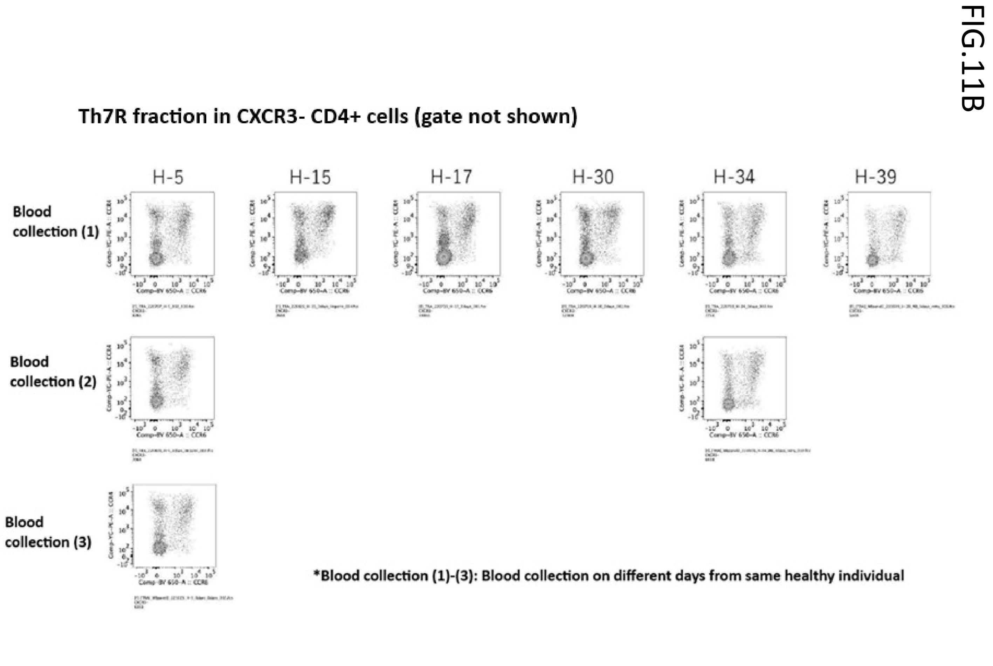

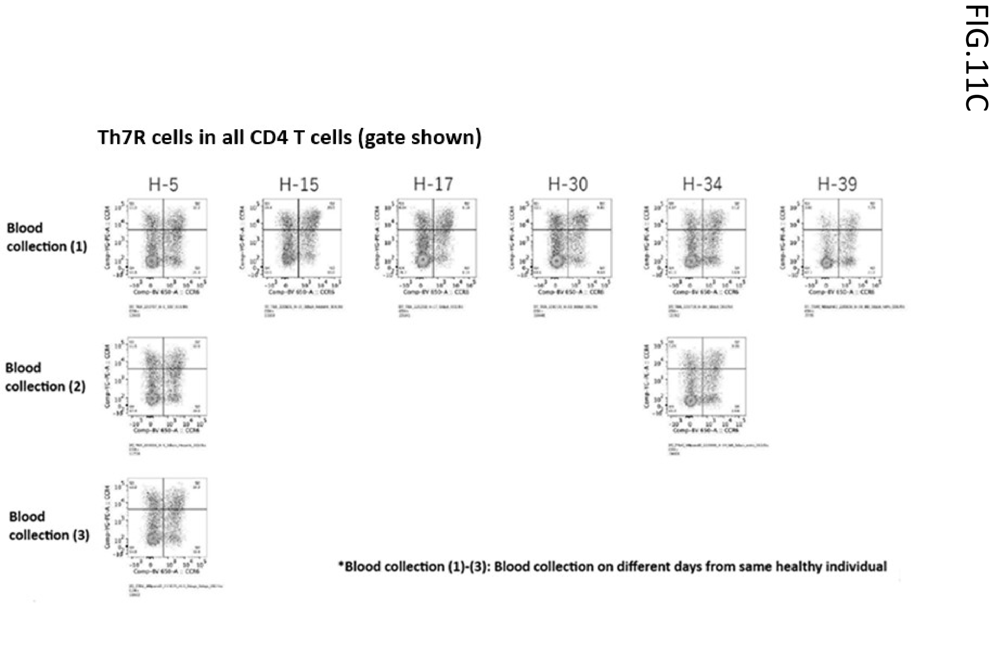

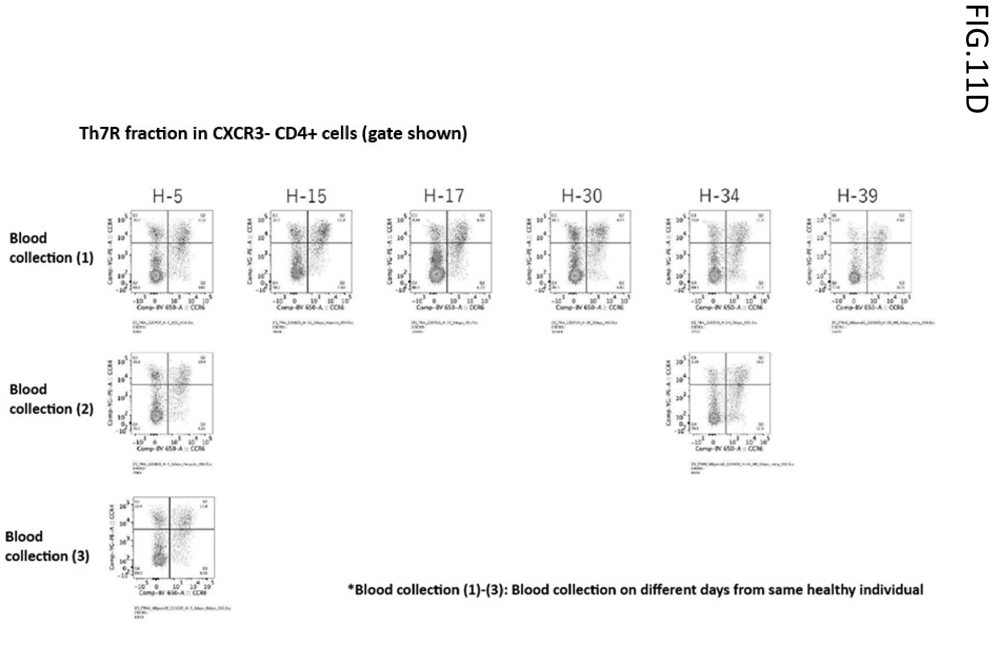

Blood was collected from six healthy individuals by using heparin blood collection tubes. Measurements were taken immediately by using WB panel 2 (Table 1). FlowJo was used for data analysis and diagram creation. The results are shown in Figures **11A to 11D.** Figure **11** shows CCR6 vs CCR4 scatter plots for CD4⁺ or CXCR3⁻ cells.
Figure **11A****:** CD4⁺ T cells; gate not shown.
Figure **11B****:** CXCR3⁻CD4⁺ T cells; gate not shown.
Figure **11C****:** same as Figure **11A****;** gate created for CXCR3⁻ is shown.
Figure **11D****:** same as Figure **11B****;** gate created for CXCR3⁻ is shown.

In view of Figure **11B****,** while dependent on the sample, CXCR3⁻CCR4^{high}CCR6⁻ cells would be generally clear as a population and separated from a population of CXCR3⁻ CCR4^{med}CCR6⁻ cells. Meanwhile, in view of Figure **11A****,** the boundary of two populations of CCR4^{high}CCR6⁻ and CCR4^{med}CCR6⁻ cells is hard to discern in the scatter plots drawn with respect to the entire CD4⁺. Thus, the gate for CCR4^{high} populations can be readily determined in Figure **11B****.** Figure **11D** is said figure with the gate determined, and the gate was also described in Figure **11C****.**

Thus, measurement of CXCR3 facilitates the creation of a gate for CCR4⁻CCR6⁺ with respect to CXCR3⁻. For this reason, it was elucidated that measurement of CXCR3 is desirable for the identification of a CCR4⁻CCR6⁺ population. This tendency is more prominent for cases using a whole blood sample than for cases using PBMCs. When three or more days have passed since blood collection, a gate for CCR4⁻CCR6⁺ with respect to CXCR3⁻ could be more readily determined for a whole blood sample than PBMCs.

### (Example 7: Measurement of self-collected sample using a micro-blood sampling device)

The experiments up to this point have revealed that whole blood can be measured even in small amounts. In this regard, this Example studied the possibility of using a micro-blood sampling device. The subjects themselves can collect blood in a simple manner with the micro-blood sampling device used in this Example.

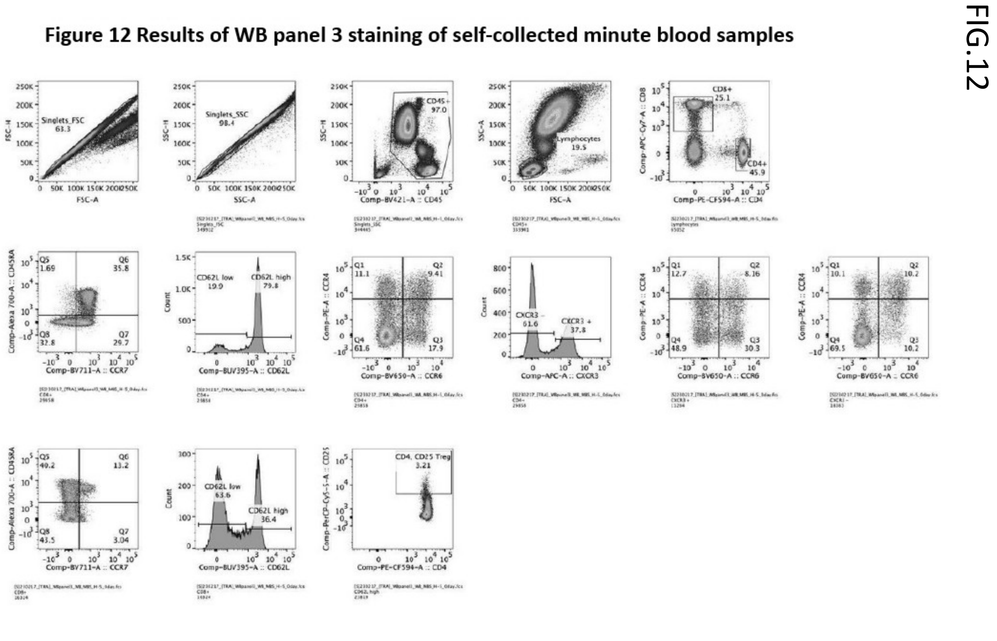

Blood was collected by puncturing the tip of the finger by using a BD Safety Lancet (Becton Dickinson) and collecting blood with an MBS capillary (heparin lithium, 100-150 µl collection line) (Micro Blood Science Inc.). Blood was immediately stained with WB panel 3 (Table 1) after collection to take measurements. Figure 12 shows the measurement results. Measurement was possible without issue, even at a very minute amount of blood collection at 100 µl to 150 µl.

It is now possible to measure a sample of 150 µl of blood (about three droplets) collected by the subjects themselves. With this approach, blood can be collected in a laboratory and immediately subjected to measurements even in the absence of a medical practitioner. Since there were panels that required culture in conventional methods using PBMCs, even if a control is suddenly needed, the need could not be met. In order to prepare a control on a daily basis, it was necessary to frequently collect about 16 ml of blood from a healthy subject. However, if a control is suddenly needed, it is sufficient to collect three droplets by using this method.

### (Comparative Example: Immobilization of cells)

Paraformaldehyde (PFA) or BD Cell FIX (solution comprising 10.0% formaldehyde, 3.55% methanol, and 0.93% sodium azide) was added to blood treated with an anticoagulant. After storage for 3 days at a predetermined temperature, PBMCs were treated then stained and subjected to measurement.

### (1) Paraformaldehyde

Blood was collected into four 4 ml blood collection tubes (Nipro, Cat. 31-764) each from two subjects (H-15 and H-34) and treated with heparin sodium. Each was dispensed into 10 tubes at 1 ml each. Two tubes were prepared by adding PBS to 4% paraformaldehyde·phosphate buffer therein for each of the final concentration of paraformaldehyde of 0.5%, 1%, 1.5%, and 2%. Nothing was added to two tubes. Each of the tubes with a concentration from 0 to 2% was incubated for 3 days at 4°C or room temperature.

Each of the tubes with a paraformaldehyde concentration from 0 to 2% was warmed to room temperature, and saline was added in an amount that is two-fold of blood. The mixtures were added to Leucosep lymphocyte separation tubes (Greiner, Cat. 163288-013)

Said tubes were centrifuged at room temperature at 800xg for 15 minutes. The top layer consisting of plasma and cells was stirred by pipetting and retrieved in a single 15 ml conical tube. To the retrieved suspension, 5% FBS/PBS was added at the same amount as the retrieved suspension or more or at 5 ml. The mixture was centrifuged at 500xg at 4°C for 5 minutes. After removing the supernatant, 10 ml of 5% FBS/PBS was added to suspend the cells. The cells were then counted.

On the same day, blood was obtained in two each of 8 ml BD Vacutainer (anticoagulant and separator-containing blood collection tube (BD, Cat. 362753)) and treated with heparin sodium. The blood was centrifuged at 2,200×g at 20°C for 20 minutes. The top layer consisting of plasma and cells was stirred by pipetting and retrieved in a single 50 ml conical tube. To the retrieved suspension, 15 ml of 5% FBS/PBS was added. The mixture was centrifuged at 500xg at 4°C for 5 minutes. After removing the supernatant, 10 ml of 5% FBS/PBS was added to suspend the cells. The cells were then counted. The blood collected in 8 ml Vacutainer was divided into a suspension for measurement on the same day and a suspension for freezing.

The suspension incubated for 3 days and suspension for measurement on the same day in 8 ml BD Vacutainer were counted and centrifuged. After removing the supernatant, 50 µl of PBS was added to suspend the cells. The suspension was transferred to a FACS tube, and a staining antibody was immediately added to the suspension, which was kept away from light for 20 minutes and stained at 4°C. Subsequently, 1 ml of 2% FBS/PBS was added to suspend the cells, and the suspension was centrifuged at 500×g at 4°C for 5 minutes. After removing the supernatant, 250 µl of 2% FBS/PBS was added to suspend the cells, and measurements were taken.

The suspension for freezing in 8 ml BD Vacutainer was centrifuged at 500×g at 4°C for 5 minutes. After removing the supernatant, Cellbanker 2 was added so that the cell concentration would be about 5.0×10⁶ cells/ml per cryotube to suspend the cells. This was dispensed into a cryotube at 1 ml each, placed in BICELL cooled to 4°C, then quickly placed in a -80°C deep freezer. On the next day, the suspension was transferred to and cryopreserved in a liquid nitrogen tank. After 2 days, the cells were melted in a thermostatic vessel held at 37°C, then suspended in 12 ml of a 10% FBS/RPMI 1640 medium (Nacalai Tesque, Cat. 30264-85), and centrifuged at 500×g at 4°C for 5 minutes, and the supernatant was removed. The cells were suspended again by adding 10 ml of 10% FBS/RPMI 1640 medium. The cells were counted, then the cellular solution was dispensed in a 24-well plate and cultured at 37°C in the presence of 5% CO₂ for 48 hours. Subsequently, the entire amount of the culture solution comprising cells was retrieved in a 15 ml conical tube, and the cells were counted. The cellular solution was then centrifuged at 500×g at 4°C for 5 minutes, suspended by adding 100 µl of PBS after removing the supernatant, and subjected to the PBMC staining step to take measurements. This was deemed as Culture PBMCs. The same applies hereinafter. The following is the list of samples.

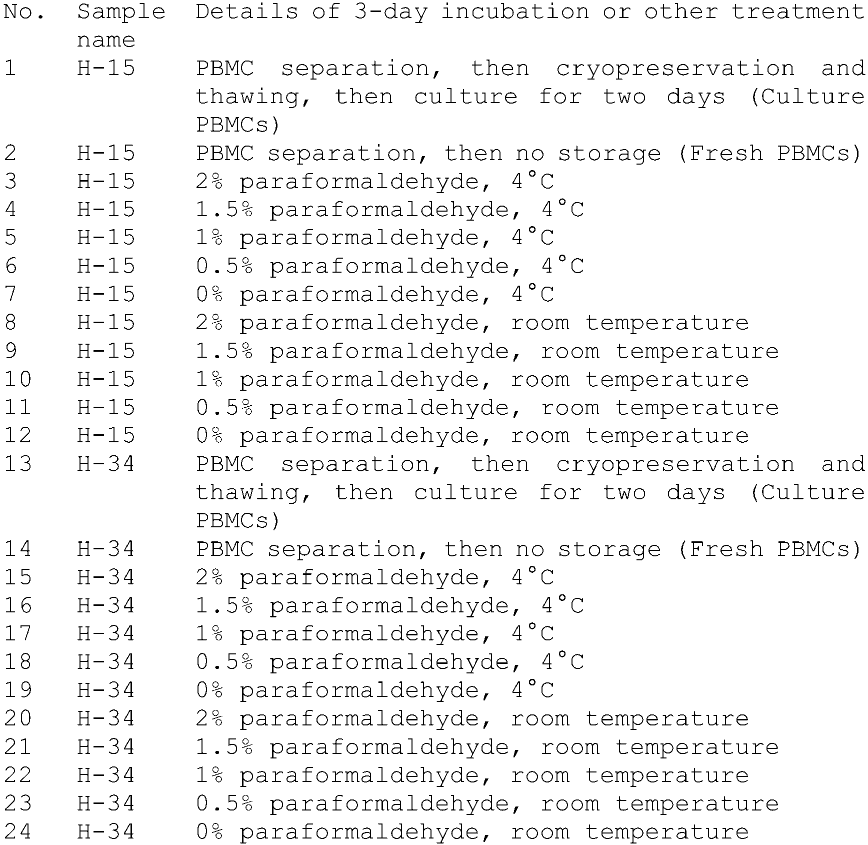

The results were as follows.

### <CD4>

### Room temperature: CD4 expression could not be confirmed for any sample with addition of PFA at a final concentration of 2-1%

Only weak expression was confirmed for samples with addition of 1%.

4°C: CD4 expression could not be confirmed for any sample with addition of PFA at a final concentration of 2-1%. Only weak expression was confirmed for samples with addition of 0.5%.

### <CXCR3>

Room temperature: Since CD4 was not expressed, a clear double peak of CXCR3 could not be confirmed.

4°C: While a double peak is manifested, the ratio of CXCR3⁺ tended to increase, with 1% as the peak, as the PFA concentration decreased to a final concentration from 2% to 0.5%, such that the expression was not stable.

### <CCR6>

Among the samples confirmed to have expression, expression of CCR6⁺ was confirmed to be much higher at 10⁴ or greater for those with addition of PFA, compared to about 10³ for those without addition of PFA, and the expression did not stabilize.

### <CCR4>

Among the samples confirmed to have expression, those with addition of PFA had a narrower range of expression with poor separation compared to those without addition of PFA.

### (2) BD Cell FIX

Blood was collected into one 4 ml blood collection tube each from two subjects (H-15 and H-34) and treated with heparin sodium. Each was dispensed into 4 tubes at 1 ml each. A solution prepared by diluting BD Cell FIX (BD, Cat. 340181: no longer on sale; currently sold as Stabilizing Fixative 3X Concentrate) with 2% FBS/PBS was added thereto so that the final dilution ratio would be x4, x10, and x20. Nothing was added to one tube. The tubes were incubated for 3 days at 4°C.

The process hereinafter is the same as that for paraformaldehyde. Specifically, PBMCs were separated. The following is the list of samples.

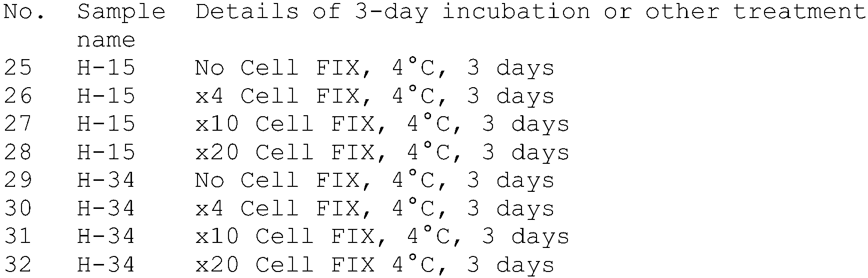

The results were as follows.

4x dilution: Lymphocyte region was not observed at all.

10x dilution: Lymphocyte region was barely observed.

20x dilution: CD4 expression was observed, but at a lower position than the general expression position. The cutoff for CXCR3 was also shifted to the left and did not stabilize.

In view of the above, when cells were immobilized by adding a stabilizer to an anticoagulant-treated blood, detection of especially CD4 and CXCR3 was problematic, so that the effective method of measuring Th7R found for whole blood samples is advantageous.

### (Example 8: Comparison of anticoagulants)

### Objective

When blood is collected at a medical setting, blood collection tubes containing EDTA as an anticoagulant are generally used in overwhelmingly many cases. In this regard, heparin sodium, EDTA, sodium citrate, and sodium fluoride were compared as anticoagulants.

### Method

Blood was collected from two healthy individuals. Two each of 2 mL blood collection tubes (only sodium citrate with the amount of blood collection of 1.8 mL) comprising each coagulant of heparin sodium (Nipro, Neotube: NP-HE0405), EDTA2k (Sekisui Medical Co., Ltd., INSEPACK II SMD520EK-murasaki-ST), sodium citrate 3.8% (Nipro, Neotube OP-CB0165-12), and sodium fluoride + EDTA-2Na (Nipro, Neotube OP-FN0205) were used and thoroughly stirred after blood collection. One tube was stored sideways at room temperature in an unopened state. The other blood collection tube was opened, and 100 µL was dispensed into a FACS tube and stored sideways in the same manner as the unopened blood collection tube. Staining antibody WB panel 3 was added to the FACS tube, and the blood was kept away from light for 15 minutes and stained at room temperature. 2 ml of hemolytic reagent (Lysing Solution, BD 349202) was then added, and the blood was kept away from light for 10 minutes, left at room temperature, and subjected to hemolysis. After centrifugation at 500×g at room temperature for 5 minutes, the supernatant was discarded. 2 ml of 2% FBS/PBS was added thereto to suspend the cells, and the cellular solution was centrifuged under the same conditions. The suspension and centrifugation were repeated again. After removing the supernatant, the cells were suspended in 250 µl of 2% FBS/PBS to take measurements with FACSymphony. 100 µL was sampled in the same manner after 1 or 2 days had passed from an opened blood collection tube and subjected to hemolysis, staining, and measurement. The unopened blood collection tube was opened after 3 days and subjected to hemolysis, staining, and measurement in the same manner as 100 µL of blood.

### Results and Discussion

The ratio of cell populations was compared for each coagulant and each number of days of storage of the sample, based on the day of blood collection with heparin sodium which had the most stable results. The results are shown in Table 5-1 to Table 5-4. Underlined numbers indicate those with an increase/decrease of 20% or more with respect to the result immediately after blood collection for heparin sodium.

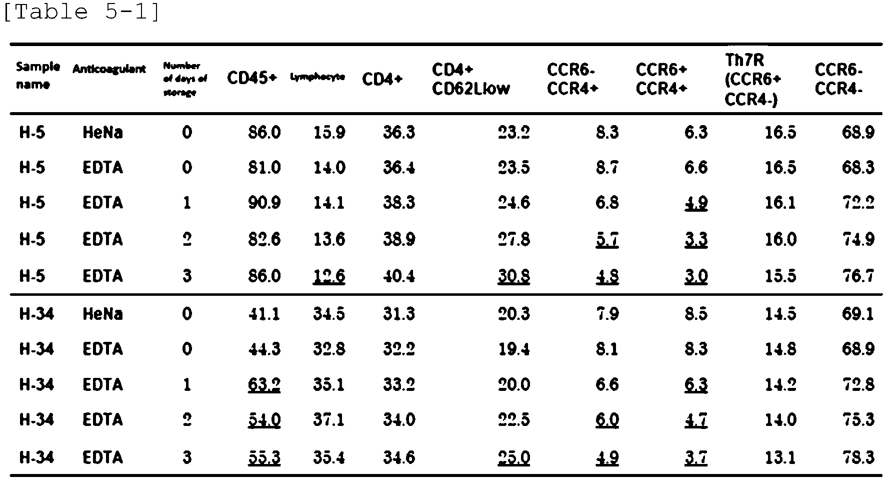

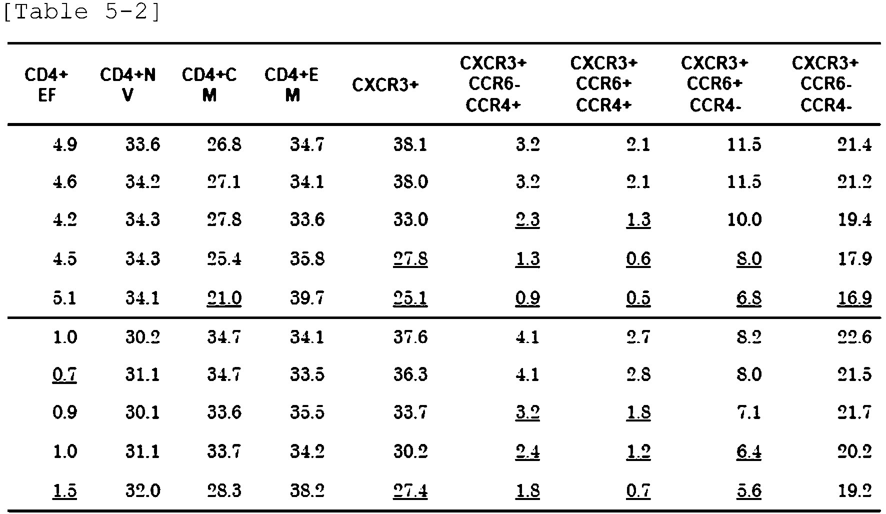

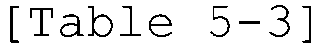

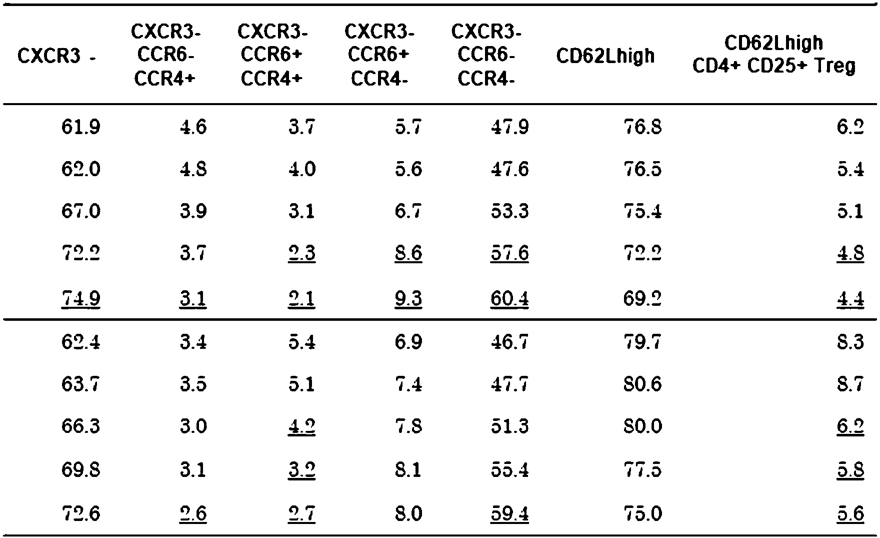

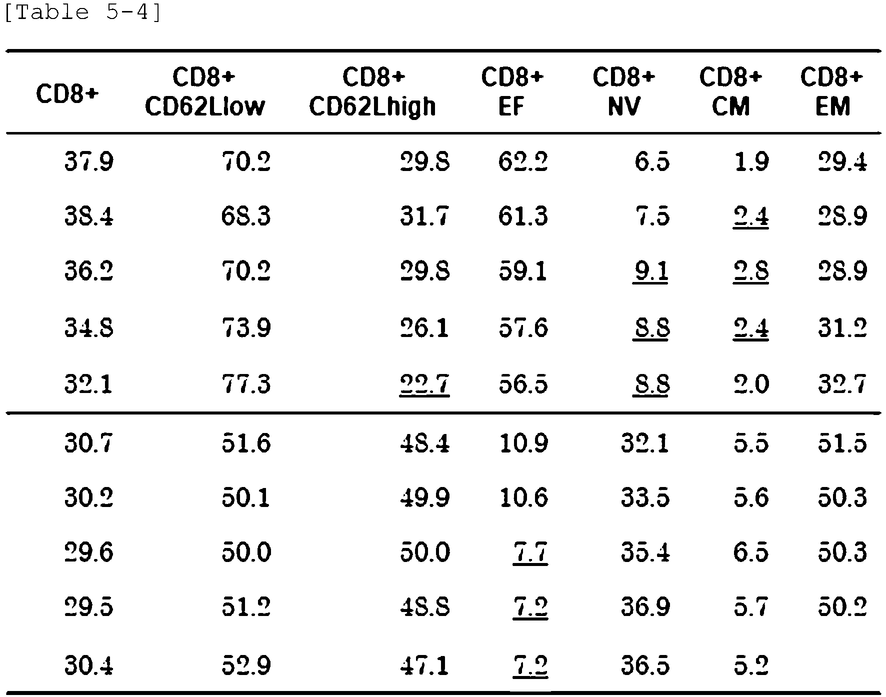

For EDTA, the ratio of CD4⁺CD62L^{low} increased and the ratio of CD4⁺CD62L^{high} decreased as the number of days of storage increased (Table 5). Further, the intensity of expression of CXCR3 decreased, the ratio of CXCR3⁻ increased, and the ratio of CXCR3⁺ decreased. However, change in the ratio of Th7R (CD4⁺CCR6⁺CCR4⁻) was small. For the measurement on the day of blood collection and on day 1, the results were similar to those for heparin sodium. However, an increase/decrease was observed upon storage, depending on the cell group. For both samples, an increase/decrease of 20% or more relative to those for the day of blood collection with heparin sodium was observed for the following cells (number of days of storage). A difference due to samples was also observed in the increase/decrease.
Increase: CD4⁺CD62L^{low} (Day 3), CXCR3⁻CCR6⁻CCR4⁻ (Day 3)
Decrease: CCR6⁻CCR4⁺ (Day 2, Day 3), CCR6⁺CCR4⁺ (Day 1, Day 2, Day 3), CXCR3⁺ (Day 3), CXCR3⁺CCR6⁻CCR4⁺ (Day 1, Day 2, Day 3), CXCR3⁺CCR6⁺CCR4⁺ (Day 1, Day 2, Day 3), CXCR3⁺CCR6⁺CCR4⁻ (Day 2, Day 3), CXCR3⁻CCR6⁻CCR4⁺ (Day 3), CXCR3⁻CCR6⁺CCR4⁺ (Day 2, Day 3), CD62L^{high}CD4⁺CD25⁺Treg (Day 2, Day 3)

For sodium citrate and sodium fluoride, an increase/decrease was observed for each cell marker (not shown). The following was found from the above results.
*The anticoagulant providing the best results was heparin sodium.
*EDTA can also be usable for measurement of Th7R.

### (Note)

As described above, the present disclosure is exemplified by the use of its preferred embodiments. It is understood that the scope of the present disclosure should be interpreted solely based on the claims. It is understood that any patent, any patent application, and any references cited herein should be incorporated herein by reference in the same manner as the contents are specifically described herein. The present application claims priority to Japanese Patent Application No. 2023-72026 filed with the JPO on April 26, 2023. The entire content thereof is incorporated herein by reference in the same manner as the contents are specifically described herein.

### [Industrial Applicability]

The present invention can be used for measuring the relative amount of a medically significant cell subpopulation in a CD4⁺ T cell population.

## Claims

1. A method of determining a relative amount of a CCR4⁻CCR6⁺ cell subpopulation in a CD4⁺ T cell population in a sample derived from a subject, comprising:
selecting a CXCR3⁻ cell subpopulation in the CD4⁺ T cell population;
setting a boundary between a CCR4⁻ cell subpopulation and a CCR4⁺ cell subpopulation in the CXCR3⁻ cell subpopulation; and
measuring a relative amount of a CCR4⁻CCR6⁺ cell subpopulation in a CD4⁺ T cell population by using the boundary.

2. The method of claim 1, wherein the sample is a whole blood sample or a peripheral blood mononuclear cell (PBMC) sample of peripheral blood of the subject.

3. The method of claim 2, wherein the sample is a whole blood sample.

4. The method of claim 3, wherein the sample has been stored for one day or longer after collection from the subject.

5. The method of claim 4, wherein the sample has been stored for one to six days after collection from the subject.

6. The method of claim 4, wherein the sample has been stored for one to three days after collection from the subject.

7. The method of claim 4, wherein the sample has been stored at about 10°C to about 40°C.

8. The method of claim 4, wherein the sample has been stored at about 12°C to about 25°C.

9. The method of claim 3, wherein the sample is about 10 µl to about 500 µl.

10. A method of determining a relative amount of a CCR4⁻CCR6⁺ cell subpopulation in a CD4⁺ T cell population in a sample derived from a subject, comprising:
preparing a whole blood sample of the subject; and
measuring a relative amount of a CCR4⁻CCR6⁺ cell subpopulation in a CD4⁺ T cell population in the whole blood sample.

11. The method of claim 10, wherein the sample has been stored for one day or longer after collection from the subject.

12. The method of claim 11, wherein the sample has been stored for one to six days after collection from the subject.

13. The method of claim 11, wherein the sample has been stored for one to three days after collection from the subject.

14. The method of claim 11, wherein the sample has been stored at about 10°C to about 40°C.

15. The method of claim 11, wherein the sample has been stored at about 12°C to about 25°C.

16. The method of claim 10, wherein the sample is about 10 µl to about 500 µl.

17. A kit for use in the method of any one of claims 1-16, comprising a detecting agent for CD4, a detecting agent for CCR4, and a detecting agent for CCR6.

18. The kit of claim 17, further comprising a detecting agent for CXCR3.

19. The kit of claim 16, wherein the detecting agent is an antibody.
